# EUROPEAN PATENT SPECIFICATION

(11) **EP 1 725 526 B1**
(45) Date of publication and mention of the grant of the patent: **12.12.2007**
(21) Application number: 05708062.4
(22) Date of filing: 28.02.2005
(51) Int. Cl.: C07D 207/22

(54) **METHOD FOR PREPARING PYRROLIDINE OXIMES**
VERFAHREN ZUR HERSTELLUNG VON PYRROLIDINOXIMEN
PROCEDE DE PREPARATION DE PYRROLIDINE OXIMES

(30) Priority: 26.02.2004 EP 04100773
(43) Date of publication of application: 29.11.2006
(73) Proprietor: Laboratoires Serono SA, 1267 Coinsins, Vaud (CH)
(72) Inventor: NADLER, William, I-00156 Roma (IT); PUPOWICZ, Doris, CH-1255 Veyrier (CH)
(74) Representative: Stevens, Ian Edward
(86) International application number: PCT/EP2005/050852
(87) International publication number: WO 2005/082848

(56) References cited:
- WO-A-01/72705
- WO-A-99/52868
- WO-A-02/102799

## Description

### Summary of the invention

The present invention is related to a new synthesis for preparing pyrrolidine oximes of general formula (I). The compounds are useful in the treatment and/or prevention of preterm labor, premature birth and dysmenorrhea.

### Field of the invention

The present invention is related to a new synthesis for preparing pyrrolidine oximes of general formula (I) : 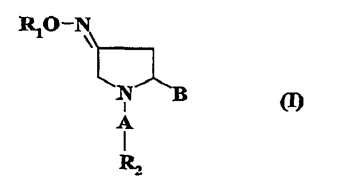 A is a carbonyl group -(C=O)-.
B is selected from the group consisting of a substituted or unsubstituted oxadiazole ring, an amido group of the formulae -(C=O)-NR₃R₄, and -(CH₂)ₙ-X-R₈,
R₁ is H or an unsubstituted or substituted C₁-C₆-alkyl. Preferably, R₁ is a methyl group.
R₂ is selected from the group comprising or consisting of unsubstituted or substituted aryl, unsubstituted or substituted heteroaryl, unsubstituted or substituted saturated or unsaturated 3-8-membered cycloalkyl. More a preferred is an aryl, in particular a phenyl group which is optionally substituted, e.g. by a further phenyl group (thus providing a biphenyl moiety).
R₃ and R₄ are independently selected from the group comprising or consisting of hydrogen, unsubstituted or substituted C₁-C₆ alkyl, unsubstituted or substituted C₂-C₆ alkenyl, unsubstituted or substituted C₂-C₆ alkynyl, unsubstituted or substituted alkoxy, unsubstituted or substituted sulfanyl, acyl, alkoxycarbonyl, aminocarbonyl, unsubstituted or substituted saturated or unsaturated 3-8-membered cycloalkyl which may contain 1 to 3 heteroatoms selected of N, O, S, unsubstituted or substituted aryl, unsubstituted or substituted heteroaryl, unsubstituted or substituted C₁-C₆-alkyl aryl, unsubstituted or substituted C₁-C₆-alkyl heteroaryl.
R₈ is selected from the group consisting of hydrogen, C₁-C₆-alkyl, C₁-C₆-alkyl aryl, heteroaryl, C₁-C₆-alkyl heteroaryl, C₂-C₆-alkenyl, C₂-C₆-alkenyl aryl, C₂-C₆-alkenyl heteroaryl, C₂-C₆-alkynyl, C₂-C₆-alkynyl aryl, C₂-C₆-alkynyl heteroaryl, C₃-C₈-cycloalkyl, heterocycloalkyl, C₁-C₆-alkyl cycloalkyl, C₁-C₆-alkyl heterocycloalkyl, C₁-C₆-alkyl carboxy, acyl, C₁-C₆-alkyl acyl, C₁-C₆-alkyl acyloxy, C₁-C₆-alkyl alkoxy, alkoxycarbonyl, C₁-C₆-alkyl alkoxycarbonyl, aminocarbonyl, C₁-C₆-alkyl aminocarbonyl, C₁-C₆-alkyl acylamino, C₁-C₆-alkyl ureido, amino, C₁-C₆-alkyl amino, sulfonyloxy, C₁-C₆-alkyl sulfonyloxy, sulfonyl, C₁-C₆-alkyl sulfonyl, sulfinyl, C₁-C₆-alkyl sulfinyl, C₁-C₆-alkyl sulfanyl and C₁-C₆-alkyl sulfonylamino;
X is selected from the group consisting of O and NR₉;
R₉ is selected from the group consisting of H, C₁-C₆-alkyl, C₁-C₆-alkyl aryl, C₁-C₆-alkyl heteroaryl, aryl and heteroaryl;
R₈ and R₉ can form together with the N atom to which they are linked to, a 5-8 membered saturated or unsaturated heterocycloalkyl ring;
n is an integer from 1 to 3.

Preferred pyrrolidine derivatives are those compounds according to formula I wherein **R₁** is a methyl group, **R₂** is a substituted or unsubstituted biphenyl.

According to one specific embodiment B is an amido group of the formula - (C=O)NHR₅, wherein R₅ is an unsubstituted or substituted C₁-C₆-alkyl aryl group, e.g. a phenylethyl group which is optionally substituted with hydrophilic moieties including amino or hydroxy.

According to a further specific embodiment, the substituent B is a 1,2,4-oxadiazole substituent which may be attached to the pyrrolidine ring according to the following modes (Xa) or (Xb):

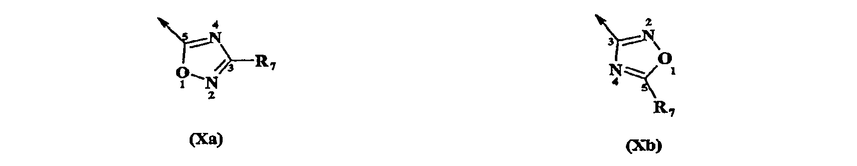

In said formulae (Xa) and (Xb), R₇ is selected from the group comprising or consisting of hydrogen, sulfonyl, amino, unsubstituted or substituted C₁-C₆-alkyl, unsubstituted or substituted C₂-C₆-alkenyl, unsubstituted or substituted C₂-C₆-alkynyl, wherein said alkyl, alkenyl, alkynyl chains may be interrupted by a heteroatom selected from N, O or S, unsubstituted or substituted aryl, unsubstituted or substituted heteroaryl, unsubstituted or substituted saturated or unsaturated 3-8-membered cycloalkyl, unsubstituted or substituted heterocycloalkyl, wherein said cycloalkyl, heterocycloalkyl, aryl or heteroaryl groups may be fused with 1-2 further cycloalkyl, heterocycloalkyl, aryl or heteroaryl group, an acyl moiety, unsubstituted or substituted C₁-C₆-alkyl aryl, unsubstituted or substituted C₁-C₆-alkyl heteroaryl, unsubstituted or substituted C₁-C₆-alkenyl aryl, unsubstituted or substituted C₁-C₆-alkenyl heteroaryl, unsubstituted or substituted C₁-C₆-alkynyl aryl, unsubstituted or substituted C₁-C₆-alkynyl heteroaryl, unsubstituted or substituted C₁-C₆-alkyl cycloalkyl, unsubstituted or substituted C₁-C₆-alkyl heterocycloalkyl, unsubstituted or substituted C₁-C₆-alkenyl cycloalkyl, unsubstituted or substituted C₁-C₆-alkenyl heterocycloalkyl, unsubstituted or substituted C₁-C₆-alkynyl cycloalkyl, unsubstituted or substituted C₁-C₆-alkynyl heterocycloalkyl, substituted or unsubstituted alkoxycarbonyl, substituted or unsubstituted aminocarbonyl , substituted or unsubstituted C₁-C₆-alkyl carboxy, substituted or unsubstituted C₁-C₆-alkyl acyl, unsubstituted or substituted C₁-C₆-alkyl acyloxy, unsubstituted or substituted C₁-C₆-alkyl alkoxy, unsubstituted or substituted C₁-C₆-alkyl alkoxycarbonyl, unsubstituted or substituted C₁-C₆-alkyl aminocarbonyl, unsubstituted or substituted C₁-C₆-alkyl acylamino, unsubstituted or substituted C₁-C₆-alkyl ureido, unsubstituted or substituted C₁-C₆-alkyl amino, unsubstituted or substituted C₁-C₆-alkyl ammonium, unsubstituted or substituted C₁-C₆-alkyl sulfonyloxy, unsubstituted or substituted C₁-C₆-alkyl sulfonyl, unsubstituted or substituted C₁-C₆-alkyl sulfinyl, unsubstituted or substituted C₁-C₆-alkyl sulfanyl, unsubstituted or substituted C₁-C₆-alkyl sulfonylamino, unsubstituted or substituted C₁-C₆-alkyl aminosulfonyl, hydroxy, halogen, cyano.

In a specific embodiment R₇ is an unsubstituted or substituted C₁-C₆-alkyl group, e.g. a methyl or an ethyl group which may optionally be substituted with hydrophilic moieties including amino or hydroxy, or R₇ is a 3 to 8 membered cycloalkyl optionally containing one or 2 heteroatoms, e.g. a pyrrolidine, furanyl, thienyl, piperidine, morpholine or piperazine.

According to a further specific embodiment, the substituent B is a group of the formulae-(CH₂)n-X-R₈, wherein. X is O, R₈ is hydrogen and n is 1.

The method employs commercially available, or easily obtainable, starting compounds.

### Background of the invention

The synthetic approach for preparing pyrrolidine oximes of formula (I) is well known. Several documents disclose the synthesis of such compounds.

WO 01/72705 for instance discloses the synthesis for the amide derivative of pyrrolidine oxime shown below (Scheme 1).

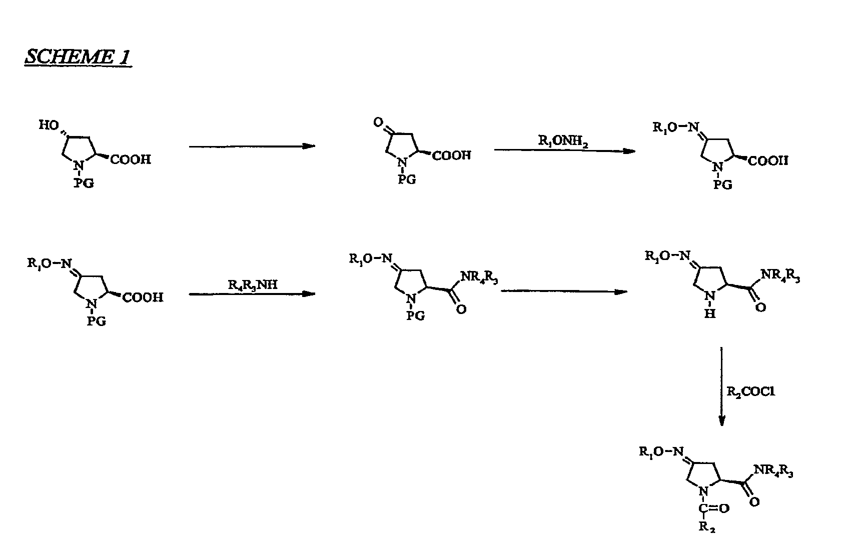

PG is a protecting group. A typical starting compound used in WO 01/72705 is Boc-protected pyrrolidine derivative (e.g. 1-(*tert*-butoxycarbonyl)-4-hydroxy-2-pyrrohdinecarboxylic acid or its follow-up product 1-(*tert*-butoxycarbonyl)4-oxo-2-pyrrolidinecarboxylic acid; cf. synthesis of intermediate 7).

A further application related to pyrrolidine derivatives is WO 04/005249. The patent application relates also to the use 1-(*tert*-butoxycarbonyl)-4-hydroxy 2-pyrrolidinecarboxylic acid as starting compound and describes the following specific pathway for synthesizing 2-hydroxyalkyl pyrrolidine oxime derivatives (see Scheme 1a). The starting material for the synthesis is again a Boo-protected pyrrolidine (e.g. 1-(*tert*-butoxycarbonyl)-4-oxo-2-pyrrolidinecarboxylic acid).

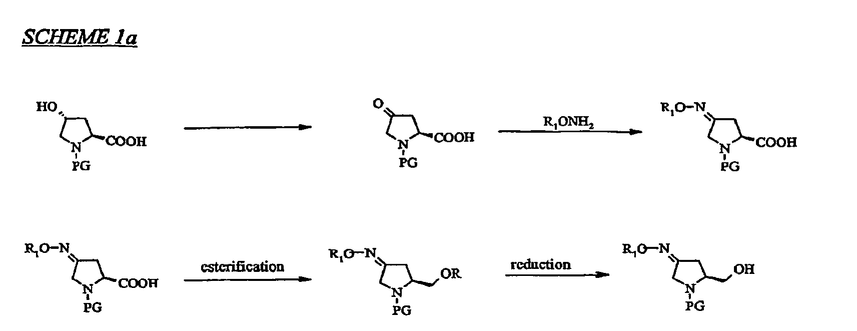

A further application related to pyrrolidine oximes is WO 02/102799. The patent application relates to the use of a protected pyrrolidine derivative as starting compound and describes the following specific pathway for synthesizing oxadiazole pyrrolidine oximes (see Scheme 2).

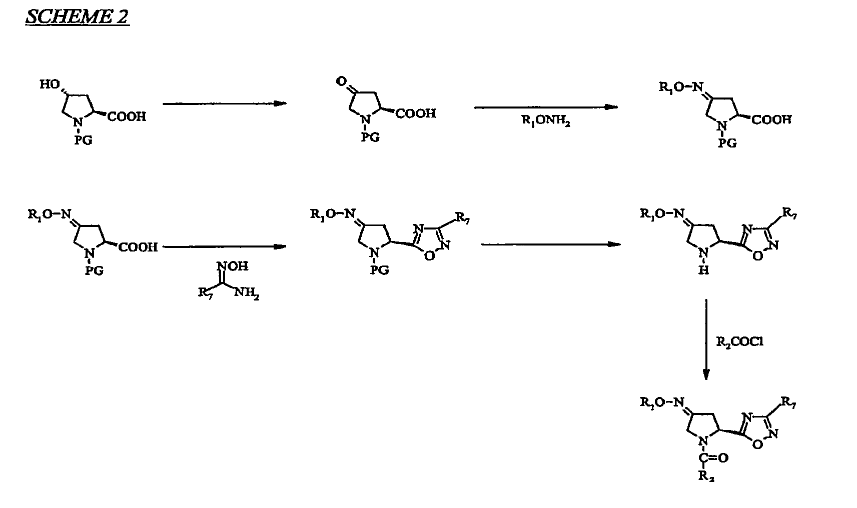

PG is a suitable protecting group. Again, the starting compound is a Boc-protected pyrrolidine (e.g. 1-(*tert*-butoxycarbonyl)-4-oxo-2-pyrrolidinecarboxylic acid).

Still a further application is WO 99/52868 (Procter & Gamble) disclosing the synthesis of hydroxamide derivatives ofpyrrolidine oxime. This pathway does not involve a protected starting compound (see scheme 3), but provides structurally different end-products (hydroxamides).

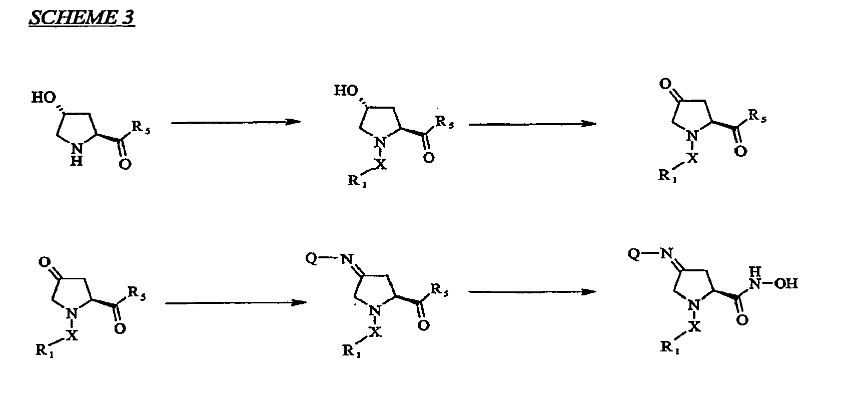

The present invention provides a new method for synthesizing pyrrolidine oxime of formula (I) that does not require the use of a Boc-protected pyrrolidine.

### Description of the invention

The present invention allows to overcome the above said problems by a synthesis that involves four steps and moreover uses, as starting compounds, compounds that can be easily synthesized or are commercially available.

The following paragraphs provide definitions of the various chemical moieties that make up the compounds according to the invention and are intended to apply uniformly through-out the specification and claims unless an otherwise expressly set out definition provides a broader definition.

"C₁-C₆ -alkyl" refers to monovalent alkyl groups having 1 to 6 carbon atoms. This term is exemplified by groups such as methyl, ethyl, n-propyl, isopropyl, n-butyl, isobutyl, *tert*-butyl, n-hexyl and the like.

"Aryl" refers to an unsaturated aromatic carbocyclic group of from 6 to 14 carbon atoms having a single ring (*eg*., phenyl) or multiple condensed rings (*e.g.*, naphthyl). Preferred aryl include phenyl, naphthyl, phenantrenyl and the like.

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, phthalazinyl, quinoxalinyl, cinnolinyl, naphthyridinyl, pyndo[3,4-b]pyridyl, pyrido[3,2 b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, phteridinyl, carbazolyl, xanthenyl or bcnzoquinolyl.

"C₃-C₈-cycloalkyl" refers to a saturated carbocyclic group of from 3 to 8 carbon atoms having a single ring (e.g., cyclohexyl) or multiple condensed rings (e.g., norbomyl). Preferred cycloalkyl include cyclopentyl, cyclohexyl, norbornyl and the like.

"C₁-C₆-alkyl cycloalkyl" refers to C₁-C₆-alkyl groups having a cycloalkyl substituent, including cyclohexylmethyl, cyclopentylpropyl, and the like.

"heterocycloalkyl" refers to a C₃-C₈-cycloalkyl group according to the definition above, in which up to 3 carbon atoms are replaced by heteroatoms chosen from the group consisting of O, S, NR, R being defined as hydrogen or methyl. Preferred heterocycloalkyl include pyrrolidine, piperidine, piperazine, 1-methylpiperazine, morpholine, and the like.

"C₁-C₆-alkyl heterocycloalkyl" refers to C₁-C₆-alkyl groups having a heterocycloalkyl substituent, including 2-(1-pyrrolidinyl)ethyl, 4-morpholinylmethyl, (1-methyl-4-piperidinyl)methyl and the like.

"C₂-C₆-alkenyl" refers to alkenyl groups preferably having from 2 to 6 carbon atoms and having one or more sites of alkenyl unsaturation. Preferred alkenyl groups include ethenyl (-CH=CH₂), n-2-propenyl (allyl, -CH₂CH=CH₂) and the like.

"C₂-C₆-alkynyl" refers to alkynyl groups preferably having from 2 to 6 carbon atoms and having one or more sites of alkynyl unsaturation. Preferred alkynyl groups include ethynyl (-C≡CH), propynyl (-CH₂C≡CH), and the like.

"Acyl" refers to the group -C(O)R where R includes "C₁-C₆-alkyl", "aryl", "heteroaryl", "C₃-C₈-cycloalkyl, "heterocycloalkyl', "C₁-C₆-alkyl aryl" or "C₁-C₆-alkyl heteroaryl".

"C₁-C₆-alkyl aminocarbonyl" refers to the group -C(O)NRR' where each R, R' includes independently hydrogen or C₁-C₆-alkyl".

"C₁-C₆-alkyl acylamino" refers to the group -NR(CO)R' where each R, R' is independently hydrogen or "C₁-C₆-alkyl".

"Halogen" refers to fluoro, chloro, bromo and iodo atoms.

"Sulfonyl" refers to a group "-SO₂-R" wherein R is selected from H, "C₁-C₆-alkyl", "C₁-C₆-alkyl" optionally substituted with halogens, such as, for example, an -SO₂-CF₃ group, "aryl", "heteroaryl", "C₁-C₆-alkyl aryl" or "C₁-C₆-alkyl heteroaryl".

"Sulfoxy" refers to a group "-S(O)-R" wherein R is selected from H, "C₁-C₆-alkyl", "C₁-C₆-alkyl" optionally substituted with halogens, such as, for example, an -SO-CF₃ group, "aryl", "heteroaryl" , "C₁-C₆-alkyl aryl" or "C₁-C₆-alkyl heteroaryl".

"Sulfinyl" refers to a group "-SO-R'R" wherein R is selected from H, "C₁-C₆-alkyl", "C₁-C₆-alkyl" optionally substituted with halogens, such as, for example, an -SO-CF₃ group, "aryl", "heteroaryl", "C₁-C₆-alkyl aryl" or "C₁-C₆-alkyl heteroaryl".

"Amino" refers to the group -NRR' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynyl-heteroaryl", "C₁-C₆-alkyl cycloalkyl", "C₁-C₆-alkyl heterocycloalkyl", and where R and R', together with the nitrogen atom to which they are attached, can optionally form a 3-8-membered heterocycloalkyl ring.

"Ureido" refers to the group -NRC(O)NR'R" where each R, R', R" is independently hydrogen, "C₁-C₆-alkyl", "C₂-C₆-alkenyl", "C₂-C₆-alkynyl", "C₃-C₈-cyoloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R' and R", together with the nitrogen atom to which they are attached, can optionally form a 3-8-membered heterocycloalkyl ring.

"Substituted or unsubstituted": Unless otherwise constrained by the definition of the individual substituent, the above set out groups, like "alkyl", "aryl" and "heteroaryl" etc. groups can optionally be substituted with from 1 to 5 substituents selected from the group consisting of"C₁-C₆-alkyl", "amino", "aryl", "heteroaryl", "sulfinyl", "sulfonyl", "alkoxy", "sulfanyl", "halogen", "carboxy", cyano, hydroxy, mercapto, nitro, and the like.

The method, according to the present invention, comprises the following 4 steps:

In accordance with the present invention, the compounds of formula (I) are prepared starting from an unprotected 4-hydroxypyrrolidinecarboxylic acid of formula (II). The compound (II) is commercially available or may be prepared according to known techniques.
**Step 1** : In a first step (cf. Scheme 4), the pyrrolidine of formula (II) is transformed into an acyl derivative of formula (IV) using a suitable acylating agent (III), e.g. an acyl chloride, an anhydride, a carboxylic acid or an ester. A preferred acylating agent is 1,1'-biphenyl-4-carbonyl chloride or 2'-methyl- 1,1'-biphenyl-4-carbonyl chloride. The preparation of such compound is disclosed for instance in WO 01/72705. 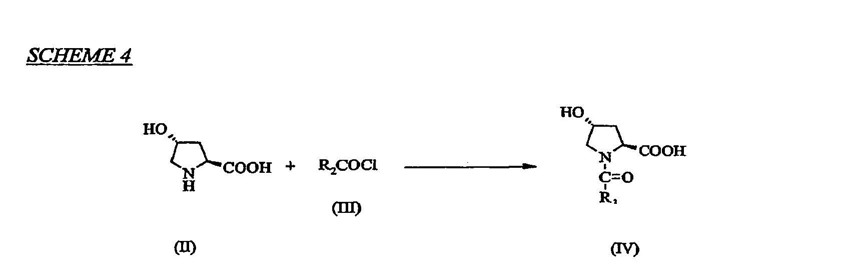 Preferably the reaction is performed in presence of a base e.g. sodium hydroxide or potassium hydroxide (Schotten-Baumann conditions) or using an organic base including triethylamine, N,N-diisopropylethylamine or pyridine.
**Step 2 :** The acyl derivative (IV) is then oxidized, with a suitable oxidizing agent, obtaining a pyrrolidone of formula (V). One suitable oxidizing agent is the pyridine-sulfurtrioxide complex (Py-SO₃) using DMSO as solvent. Preferably, the reaction is performed in presence of triethylamine.
   Additional examples for suitable oxidizing reagents include e.g. oxalyl chloride/DMSO, trifluoroacetic acid anhydride/DMSO, dicyclohexyl carbodiimide/DMSO, pyridinium dichromate, pyridinium chlorochromate, Jone's oxidation or the Dess-Martinperiodinane 1,1,1-tris(acetyloxy)-1-λ⁵, 2-benziodoxol-3(1H)-one. 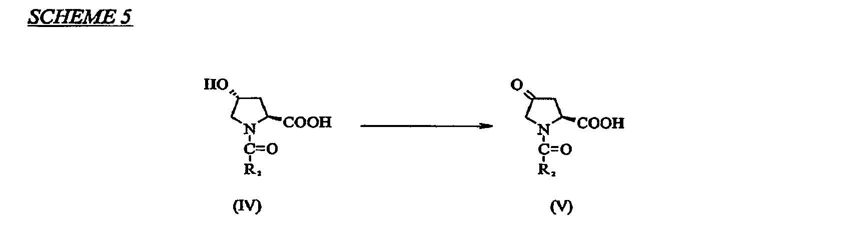
**Step 3** : Then the compound of formula (V) is transformed into compound (VII) using a suitable alkoxylamine, aryloxylamine or hydroxylamine of general formula (VI), e.g. O-methylhydroxylamine hydrochloride(such compound is commercially available) in the presence of an organic base, such as triethylamine or N,N-diisopropylethylamine.. 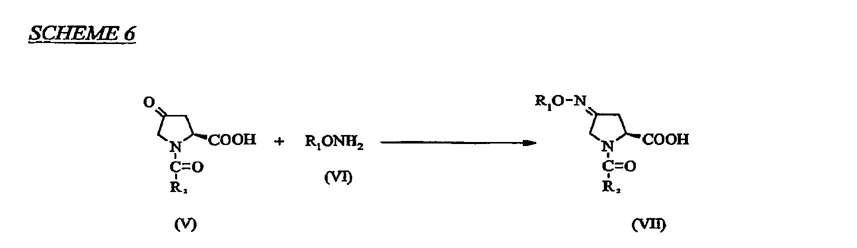
**Step 4** : The compound (VII) is then transformed into either of the compounds (Ia) or (Ib) using either an amine of general formula (VIII) or an N-hydroxyamidoxime of general formula (IX). The preparation of N-hydroxyamidoxime of general formula (IX) is disclosed for instance in WO 02/102799.

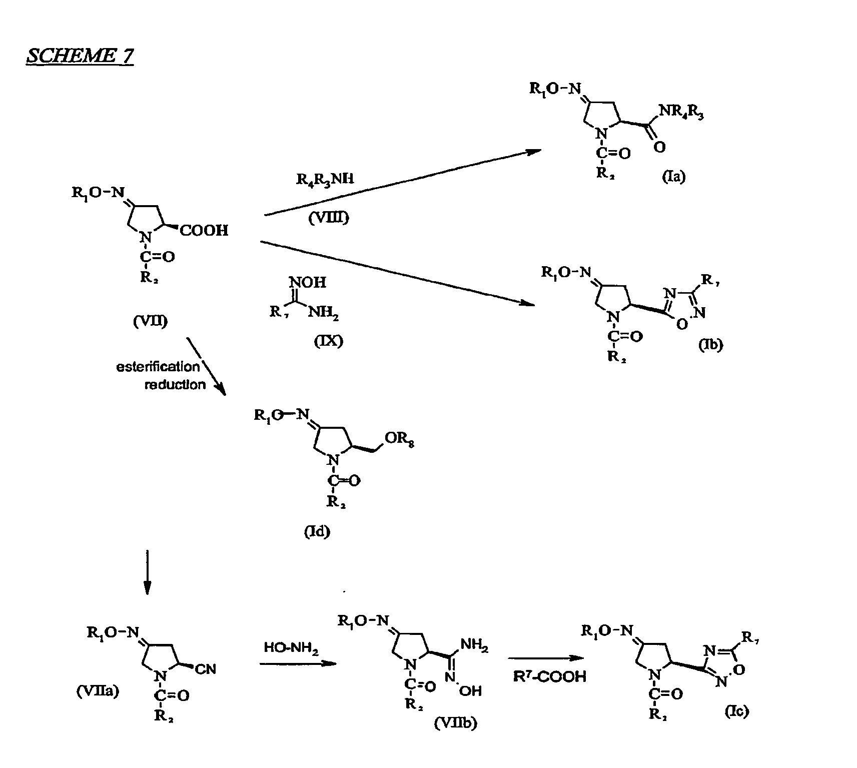

In the case that the final product (Ic) is to be generated, Step 4 has to be adjusted in the sense that first a N-hydroxyamidoxime) (VIIb) is to be provided by transforming compound (VII) into a nitrile (VIIa) (e.g. directly form the acid (this is known in the literature) or via an amide) which is then further reacted with a carboxylic acid of formula R⁷-COOH or e.g. the corresponding acyl chloride to finally yield compound (Ic) after heating of the intermediate product e.g. with an excess of pyridine. Preferably, coupling agents are used for the reaction of amidoxime (VIIb) with the carboxylic acid, e.g. N-(3-dimethylaminopropyl)-N'-ethylcarbodiimide hydrochloride, carbonyldiimidazole, dicyclobexylcarbodiimide, pivaloyl chloride, isobutyl chloroformate (or any other of the usual reagents known for peptide bond formation).

In the case that the final product (Id) is to be generated, wherein R₆ is hydrogen various well-known esterification and reduction agents can be used in order to convert the carboxy group to a hydroxyalkyl group. Examples for esterification agents are dimethylsulphate, methyl iodide, methyl tosylate, diazomethane derivatives, such as trimethylsilyl diazomethane, which are all esterification reagents that work under slightly basic or neutral conditions. Examples for reduction agents are lithium borohydride, lithium aluminum hydride, sodium-bis(2-methoxyethoxy)aluminumhydride (Red-Al), diisobutylaluminum hydride (DIBAL) and the like.

The final products of formulae (Ia), (Ib), (Ic) and (Id) may be further transformed, in particular in respect to the moiety R¹, R², R⁷ and R⁸. Thus, a final product (Ic) wherein R⁷ contains a functional group, said moiety may be transformed to another moiety by suitable means, including hydrolysis, esterification, saponification, alkylation etc. Also, the compounds of the invention may be subjected to further purification steps, including chromatography and re-crystallization.

The new synthetic approach for preparing the compound of formula (I) does not involve the use of the relatively expensive Boc-protected pyrrolidine but from cheap and easily available 3-hydroxyproline.

A further advantage of the new synthetic approach concerns the preparation of compounds having polar moieties attached to the 2-carboxamide or the 2-oxadiazole position (for instance R³, R⁴, R⁷ being a moiety (e.g. an alkyl or aryl) that contains e.g. a hydroxy or amino substituent, including a cyclic amine). The present new method avoids a final N-capping step (as seen in Scheme 2), implying the use of a nucleophile (e.g. acyl chloride) that may choose between the pyrrolidine amine and said second polar moiety, e.g. a hydroxy or amino substituent, to react.

In one embodiment, the new synthetic approach for preparing may be employed for the industrial manufacturing of the compounds of formula (I).

The present invention shall be illustrated by means of the following examples.

### Example 1 : Preparation of (2S,4E and 4Z)-N-[(2S)-2-hydroxy-2-phenylethyl]-4-(methoxyimino)-1-[(2'-methyl[1,1'biphenyl]-4-yl)carbonyl]-2-pyrrolidine carboxamide

### Step 1 : Preparation of (4R)-4-hydroxy-1-[(2'-methyl1,1'-biphenyl-4-yl)-carbonyl]-L-proline (compound (IV) in scheme 4)

4-Hydroxy-*L*-proline (0.625wt) and water (3.3vol) are charged to a 20L flange flask. Triethylamine (2.42vol) was added to the contents dropwise such that the temperature is maintained in the range 10 to 20°C. Tetrahydrofuran (5.0vol) was added and the reaction mixture was cooled to 0 to 5°C. 2'-methyl-1,1'-biphenyl-4-carboxylic acid chloride, 1.0wt) and tetrahydrofuran (5.0vol) were charged to a separate flask, stirred for 5 to 10 minutes and then added to the reaction mixture ensuring that the temperature was maintained in the range 0 to 10°C. The reaction mixture was warmed to 15 to 25°C over 60-120 minutes and maintained at 15 to 25°C until reaction completion was noted by TLC analysis. The resultant is concentrated under vacuum at 35 to 40°C, water (10.0vol) and ethyl acetate (5.0vol) are added to the residue and the contents stirred for 5 to 10 minutes. The layers were separated, the aqueous phase acidified to pH1 with aqueous hydrochloric acid (6M, approx. 3.0vol) and the resulting slurry cooled to and aged at 0 to 10°C for 25 to 40 minutes. The precipitate was collected by filtration, the isolated solid transferred to a suitable flange flask and slurried in warm (35 to 60°C) water (5.0vol) for 10 to 25 minutes. The solid was collected by filtration and the hot water slurry treatment was repeated as above. After the second slurry treatment the solid was azeotropically dried with toluene (2x 5.0vol) at 40 to 50°C. Ethyl acetate (2.5vol) and heptanes (2.5vol) were added to the residue, the resulting slurry cooled to and aged 0 to 5°C for 30 to 40 minutes, filtered, the collected solids washed with pre-cooled (0 to 5°C) ethyl acetate:heptanes (1:1, 2.0vol) and dried under vacuum at 30 to 40°C to constant weight to give (4R)-4-hydroxy-1-[(2'-methyl-1,1'-biphenyl-4-yl)carbonyl]-*L*-proline as a white solid. Yield: 85.9%.

### Step 2: Preparation of 1-[(2'-methyl-1,1'biphenyl-4-yl)carbonyl]-4-oxo-L-proline (compound (V) in scheme 5)

(4*R*)-4-Hydroxy-1-[(2'-metlzyl-1,1'-biphenyl-4-yl)carbonyl]-*L*-proline (product of Step 1, 1.0wt) and dimethyl sulphoxide (2.5vol) were charged to a 20L flange flask. The contents were heated to 35 to 40°C and maintained at this temperature until complete dissolution was achieved. The solution was cooled to 5 to 10°C under a nitrogen atmosphere and triethylamine (3.0vol) was added such that the temperature was maintained in the range 5 to 20°C. Pyridine-sulphur trioxide complex (1.47wt) and dimethyl sulphoxide (4.9vol) were charged to a separate flask, stirred for 5 to 10 minutes and then added to the reaction mixture such that the temperature was maintained in the range 15 to 25°C. The reaction was stirred at 15 to 25°C until reaction completion is noted by HPLC analysis (typically 1 to 3 hours). The vessel contents were cooled to 0 to 10°C and quenched with aq. hydrochloric acid (3M, 8vol) maintaining the temperature below 30°C. Tetrahydrofuran (5.0vol) and heptanes (1.0vol) were then added, the layers separated, the aqueous phase extracted with tetrahydrofuran (2x 5.0vol) and the combined organics washed with aq. hydrochloric acid (1M, 2x 2.0vol) and saturated brine solution (2x 2.0vol). The aqueous washes were combined and back-extracted with tetrahydrofuran (2x 1.0vol), the organics combined, dried over magnesium sulphate (3wt) and filtered. The filter-cake was washed with tetrahydrofuran (1.0vol) and the filtrates are concentrated under vacuum at 40 to 45°C to give a pale brown foam. Ethyl acetate (10.0vol) was added to the residue, the contents stirred for 5 to 10 minutes and the solvent removed under vacuum at 40 to 45°C. The residue was transferred to a flask, ethyl acetate (8.0vol) was added and the contents were heated to reflux. A slurry of activated carbon (0.14wt) in ethyl acetate (5.0vol) was added and reflux conditions reestablished and maintained for 20 to 30 minutes. The contents were cooled to 40 to 45°C, filtered, the filter-cake was washed with ethyl acetate (2.5vol) and the filtrates concentrated to 2.5 to 3.0vol under vacuum at 40 to 45°C. The slurry was diluted with ethyl acetate (0.5vol) and heated to reflux. Heptane (3.0vol) was added and the contents allowed to cool to 15 to 25°C over 1 to 2 hours. The slurry was further cooled to at 0 to 5°C for 2 to 3 hours, filtered and the filter-cake washed with ethyl acetate:heptane [(1:1), 1.0vol] pre-cooled to 0 to 5°C followed by heptane (5.0vol). The isolated solid was dried under vacuum at 40 to 45°C to give 1-[(2'-methyl- 1,1'-biphenyl-4-yl)carbonyl]-4-oxo-*L*-proline as an off-white solid. Yield: 60.3%.

### Step 3: Preparation of 4-methoxyimino-1-[(2'-methyl-1,1'-biphenyl-4-yl)-carbonyl]-L-proline (compound (VII) in scheme 6)

1-[(2'-Methyl-1,1'-biphenyl-4-yl)carbonyl]-4-oxo-*L*-proline (of Step 2, 1.0wt), *O-*methyl-hydroxylamine hydrochloride (0.285wt) and dichloromethane (20vol) were charged to a 20L flange flask and cooled to 0 to 5°C. Triethylamine (0.91vol) was charged to the flask such that the temperature was maintained in the range 0 to 10°C, the reaction mixture was warmed to 15 to 25°C and maintained within this temperature range for 16 to 20 hours. The reaction mixture was concentrated under vacuum at 40 to 45°C, the residue dissolved in ethyl acetate (10.0vol) and washed with aq. hydrochloric acid (1M, 2x 5.0vol). The aqueous washes were combined and back extracted with ethyl acetate (5.0vol), the organic extracts combined and washed with satuarted brine solution (10.0vol), dried over magnesium sulphate (0.5wt), filtered and the filter-cake washed with ethyl acetate (5.0vol). The filtrates were concentrated under vacuum at 40 to 45°C to give 4-methoxyimino-1-[(2'-methyl-1,1'-biphenyl-4-yl)carbonyl]-*L*-proline in the expected *E:Z* mixture. Yield: 95.6%.

### Step 4 : Preparation of N-[2-hydroxy-2-phenylethyl]4-(methoxyimino)-1-[(2'-methyl[1,1'-biphenyl]-4-yl)carbonyl]-2-pyrrolidine carboxamide (compound (Ia) in scheme 7)

4-Methoxyimino-1-[(2'-methyl-1,1'-biphenyl-4-yl)carbonyl]-*L*-proline (of Step 3, 1.0wt) and dichloromethane (10.0vol) were charged to a 20L flange flask and cooled to 0 to 5°C under an atmosphere of nitrogen. *N*-Methylmorpholine (0.78vol) was added at 0 to 5°C followed by pivaloyl chloride (0.37vol) at 0 to 5°C. The vessel contents were stirred at 0 to 5°C until the formation of the mixed anhydride was complete (typically 30 to 60 minutes). To a separate 20L flange flask is charged *(*S)-2-amino-1-phenylethanol (0.47wt, 1.2eq.) and dichloromethane (3.0vol) and the resultant was stirred for 5 to 25 minutes. The solution was then cooled to 10 to 15°C and was charged with the mixed anhydride such that the temperature was maintained at 5 to 15°C. The reaction mixture was warmed to 15 to 25°C and maintained within this temperature range until reaction completion is noted by HPLC analysis. The resultant was concentrated under vacuum at 35 to 45°C, the residue partitioned between *tert*-butyl methyl ether (TBME, 10.0vol) and aq. citric acid solution (0.1M, 5.0vol), the layers separated and the organic phase further was washed with aq. citric acid solution (0.1M, 2x 5.0vol), sat. aq. sodium hydrogen carbonate solution (2x 5.0vol) and sat. brine solution (5.0vol). The organic phase was dried over magnesium sulphate (1wt), filtered and the filter-cake was washed with TBME (2.0vol). The filtrates were concentrated under vacuum at 35 to 45°C to give a brown semi-solid. Dichloromethane (5.0vol) was added to the residue and the contents were concentrated under vacuum at 35 to 45°C to a gum. The process was repeated with a further portion of dichloromethane (1.0vol) and a crude end product was obtained as the expected E:Z mixture. Yield: 84.4%

### Example 2: (3E,5S)-1-([1,1'-biphenyl]-4-ylcarbonyl)-5-[3-(2-hydroxyethyl)-1,2,4-oxadiazol-5-yl]-3-pyrrolidinone O-methyloxime; (3Z,5S)-1-([1,1'-biphenyl]-4-ylcarbonyl)-5-[3-(2-hydroxyethyl)-1,2,4-oxadiazol-5-yl]-3-pyrrolidinone O-methyloxime

### Step 1: Preparation of (2S,4R)-1-(biphenyl-4-ylcarbonyl)-4-hydroxy-pyrrolidine-2-carboxylic acid (compound (IV) in scheme 4)

4-Hydroxy-*L*-proline (0.670Kg, 5.11mol, 0.67wt), tetrahydrofuran (5.00L, 5.0vol) and water (3.30L, 3.3vol) were charged to a 20L flange flask. Triethylamine (2.570L, 2.57vol) was added dropwise such that the temperature was maintained in the range 10 to 15°C and the resultant cooled to 0 to 5°C. 1,1'-Biphenyl-4-carbonyl chloride (1.00Kg, 3.78mol, 1.0wt) and tetrahydrofuran (5.00L, 5.0vol) were charged to a separate flask, stirred as a slurry for 5 to 10 minutes and added to the reaction mixture over 40 to 50 minutes ensuring that the temperature was maintained in the range of 0 to 10°C. The reaction mixture was heated to 15 to 25°C over 60 to 120 minutes and maintained at 15 to 25°C until reaction completion was noted by TLC analysis (dichloromethane:methanol:acetic acid 90:10:1; visualisation UV; product R_{f} 0.13). The reaction mixture was concentrated under reduced pressure at 35 to 40°C, water (8.00L, 8.0vol) and ethyl acetate (5.00L, 5.0vol) added to the residue and the contents stirred for 5 to 10 minutes. The layers were separated, the aqueous phase acidified to pH1 with rapid addition of aqueous hydrochloric acid (6M, approx. 900mL, 0.9vol) and the resulting slurry cooled to 0 to 10°C for 40 to 50 minutes. The precipitate was collected by filtration, the isolated solids slurried in warm water (35 to 60°C, 5.00L, 5.0vol) for 10 to 25 minutes and the solids collected by filtration. The warm water slurry treatment was repeated as above. The collected solids were combined with those from an equally sized batch, charged to a 20L flange flask, acetone (10.00L, 5.0vol) added and the reaction mixture heated to and maintained at reflux (approx. 65°C) for 10 to 20 minutes. The resultant was allowed to cool to 15 to 25°C, stirred at 15 to 25°C for 12 to 18 hours and further cooled to and aged at 0 to 5°C for 60 minutes. The precipitate was collected by filtration and washed with ethyl acetate:acetone (1:1, 4.00L, 2vol). The solids were pulled dry on the filter and further dried under vacuum at 40 to 45°C to constant weight to give (2*S*,4*R*)-1-(biphenyl-4-ylcarbonyl)-4-hydroxy-pyrrolidine-2-carboxylic acid as a beige solid. The filtrates were concentrated to approximately 3.00L under reduced pressure to afford a second crop of material which was collected by filtration, washed with ethyl acetate:heptanes (1:1, 2x 4.00L, 2x 2vol) and pulled dry on the filter. Drying under vacuum at 40 to 45°C to a constant weight gave the title compound as a beige solid. Total output: 2.616Kg, Yield: 91.9%).

### Step 2: Preparation of (2S)-1-(biphenyl-4-ylcarbonyl)-4-oxo-pyrrolidine-2-carboxylic acid (compound (V) in scheme 5)

(2*S*, 4*R*)-1-(Biphenyl-4-ylcarbonyl)-4-hydroxypyrrolidine-2-carboxylic acid (0.806Kg, 1.0wt) and dimethyl sulfoxide (5.00L, 6.25vol) were charged to a 20L flange flask and stirred under nitrogen until complete dissolution was achieved The solution was cooled to 10 to 15°C and triethylamine (2.40L, 3.0vol) was added such that the internal temperature was maintained in the range 10 to 20°C. Pyridine-sulfur trioxide complex (1.224Kg, 1.53wt) was charged to the reaction mixture portion-wise such that the internal temperature was maintained in the range 10 to 25°C. Stirring at 15 to 25°C was continued until reaction completion was noted by TLC analysis (dichloromethane:methanol:acetic acid 90:10:1; product R_{f} 0.28), typically within 1 to 3h. The reaction mixture was cooled to 0 to 10°C and quenched with aq. hydrochloric acid (3M, 6.460L, 8.0vol) maintaining the temperature below 30°C. Tetrahydrofuran (2.00L, 2.5vol) and ethyl acetate (2.00L, 2.5vol) were added, the layers separated, the aqueous phase extracted with tetrahydrofuran:ethyl acetate (1:1, 4.00L, 5.0vol) and the combined extracts washed with aq. hydrochloric acid (1M, 2x 1.60L, 2x 2.0vol) and sat. brine solution (1.60L, 2.0vol). Activated carbon (160g, 0.2wt) was charged to the organic phase and the resulting slurry heated to and maintained at reflux (65 to 70°C) for 0.5h. The reaction mixture was cooled to 20 to 30°C, magnesium sulphate (375g, 0.5wt) charged, stirring maintained for 10 minutes the mixture filtered through celite. The collected solids were washed with ethyl acetate (2x 0.800L, 2x 1.0vol) and the combined filtrates concentrated under reduced pressure at 40 to 45°C to give the title compound (2*S*)-1-(biphenyl-4-ylcarbonyl)-4-oxo-pyrrolidine-2-carboxylic acid as a viscous, orange oil (0.769Kg, Yield: 96.0%). The material was used in the next step without further purification.

### Step 3: Preparation of (2S)-1-(biphenyl-4-ylcarbonyl)-4-(methoxyimino)-pyrrolidine-2-carboxylic acid (compound (VII) in scheme 6)

Crude (2S)-1-(biphenyl-4-ylcarbonyl)-4-oxopyrrolidine-2-carboxylic acid (1.550Kg, 5.01mol, 1.0wt), *O*-methylhydroxylamine hydrochloride (0.620Kg, 7.42mol, 0.40wt) and dichloromethane (12.40L, 8.0vol) were charged to a 20L flange flask and cooled to 0 to 5°C. Triethylamine (1.752L, 1.13vol) was added to the reaction mixture over 45 to 60 minutes such that the internal temperature was maintained in the range 0 to 10°C. The reaction mixture was warmed to 15 to 25°C and maintained in this temperature range until reaction completion (typically 12 to 18 hours) was noted by TLC analysis (dichloromethane:methanol:acetic acid 90:10:1, visualisation UV; product R_{f} 0.27, 0.35 Z, *E*)*.* The reaction mixture was concentrated under reduced pressure at 40 to 45°C, the residue dissolved in ethyl acetate (12.40L, 8.0vol) and washed with aq. hydrochloric acid (2M, 2x 4.650L, 2x 3.0vol). The aqueous washes were combined and back extracted with ethyl acetate (4.650L, 3.0vol). The organic extracts were combined, washed with sat. brine solution (4.650L, 3.0vol), dried over magnesium sulphate (770g, 0.5wt), filtered and the filter-cake washed with ethyl acetate (4.650L, 3.0vol). The filtrates were concentrated under reduced pressure at 40 to 45°C to give a beige solid. The crude product was slurried in ethyl acetate (3.10L, 2.0vol) at 15 to 20°C, cyclohexane (12.40L, 8.0vol) added over 15 minutes and the resulting slurry cooled to and aged at 0 to 5°C for 1h. The precipitate was collected by filtration, washed with ethyl acetate:cyclohexane (1:2; 4.650L, 3.0vol) and dried under vacuum at 40 to 45°C to constant weight to afford the title product as a beige solid (1.132Kg, Yield: 66.8%).
The isolation filtrates (from 9 runs of the above reaction) were combined and concentrated under reduced pressure at 40 to 45°C. The residue (approximately 1.00Kg) was hot slurried (70 to 75°C) in ethyl acetate (7.00L), cooled to and aged at 0 to 5°C for 2 hours, filtered and the collected solids dried under vacuum at 40 to 45°C to constant weight to provide a second crop of (2*S*)-1-(biphenyl-4-ylcarbonyl)-4-(methoxyimino)pyrrolidine-2-carboxylic acid (0.732Kg, 4.9%th).

### Step 4a: Preparation of (2S)-1-(biphenyl-4-carbonyl)-5-[3-(2-triethylsilanyloxyethyl)-1,2,4-oxadiazol-5-yl]-pyrrolidin-3-one-O-methyloxime (compound (Ib) in scheme 7)

(2*S*)-1-(Biphenyl-4-ylcarbonyl)-4-(methoxyimino)pyrrolidine-2-carboxylic acid (0.560Kg, 1.0wt) and tetrahydrofuran (8.40L, 15.0vol) were charged to a 20L flange flask and cooled to 0 to 5°C. Carbonyl diimidazole (0.280Kg, 0.5wt) was added portion-wise such that the internal temperature was maintained in the range 0 to 10°C. The reaction mixture was warmed to and stirred at 15 to 20°C until reaction completion (1 to 2h) was noted by TLC analysis (ethyl acetate, visualisation UV). N-Hydroxy-3-triethylsilanyl-oxypropionamidine (0.381Kg, 0.68wt, 1.0eq. corrected for silanol content) as a solution in tetrahydrofuran (2.80L, 5.0vol) was then added in one portion and stirring continued at 15 to 25°C with reaction monitoring by TLC analysis (ethyl acetate, visualisation UV). Reaction completion was noted after 1 hour. The reaction mixture was concentrated under reduced pressure at 40 to 45°C and the residue combined with two batches of similar input. Pyridine (5.040L, 3vol) was added to the combined material and the resultant heated to and maintained at 85 to 90°C until HPLC analysis indicated complete cyclisation. The reaction mixture was concentrated under reduced pressure at 40 to 45°C, the dark oily residue treated with ethyl acetate (16.80L, 10vol) and washed with 25% aq. citric acid solution (3x 5.00L, 3x 3.Ovol). The aqueous extracts were combined and back-extracted with ethyl acetate (5.00L, 3vol), the combined organics washed with brine (5.00L, 3.Ovol), dried over magnesium sulphate (1.680Kg, 1wt), filtered and the filter-cake washed with ethyl acetate (1.70L). The combined filtrates were concentrated under reduced pressure at 40 to 45°C to yield crude (2*S*)-1-(biphenyl-4-carbonyl)-5-[3-(2-triethylsilanyloxyethyl)-1,2,4-oxadiazol-5-yl]pyrrolidin-3-ono-*O*-methyloxime as a brown oil which was used without further purification (2.796Kg, 108%).

### Step 4b: Preparation of (2S)-1-(biphenyl-4-carbonyl)-5-[3-(2-hydroxyethyl)-1,2,4-oxadiazol-5-yl]pyrrolidin-3-ono-O-methyloxime

Crude (2*S*)-1-(biphenyl-4-carbonyl)-5-[3-(2-triethylsilanyloxyethyl)-1,2,4-oxadiazol-5-yl]-pyrrolidin-3-one-*O*-methyloxime (1.398Kg, 1.0wt) as a solution in tetrahydrofuran (6.990L, 5.0vol) was treated with a 1% solution of trifluoroacetic acid in water (3.495L, 2.5vol). TLC analysis (ethyl acetate; visualisation UV; product R_{f} 0.35, 0.48 Z, E) indicated reaction completion after 30 minutes. The pH of the reaction mixture was adjusted to pH 7 with sat. aq. sodium hydrogen carbonate solution (1.00L, 0.72vol) and ethyl acetate (6.990L, 5vol) charged. The layers were separated, the organic phase washed with sat. aq. sodium hydrogen carbonate solution (2.796L, 2.0vol), the aqueous washes combined and back-extracted with ethyl acetate (2.796L, 2.0vol). The organics were combined, washed with brine (4.794L, 3vol), dried over magnesium sulphate (1.164Kg, 0.75wt), filtered and the filter-cake washed with ethyl acetate (2x 0.699L, 2x 0.5vol). The combined filtrates were concentrated under reduced pressure at 40 to 45°C to give an oily residue which was combined with the residue from a second batch of similar input. Total crude: 2.592Kg. The crude material was dissolved in acetonitrile (2.592L, 1vol), heptanes (26.00L, 10vol) charged and the resultant heated to and maintained at 45 to 55°C for 30 minutes. The lower acetonitrile phase was separated, charged to vigorously stirred *t*-butyl methyl ether (56.00L, 22vol), the mixture cooled to and aged at 0 to 5°C for 1 to 2 hours, filtered and concentrated under reduced pressure at 40 to 45°C to give the title compound as a pale yellow solid (2.037Kg, 93.3%).

### Example 2a: (3E,5S)-5-[3-(2-hydroxyethyl)-1,2,4-oxadiazol-5-yl]-1-[(2'-methylbiphenyl-4-yl)carbonyl]pyrrolidin-3-one O-methyloxime: (3Z,5S)-5-[3-(2-hydroxyethyl)-1,2,4-oxadiazol-5-yl]-1-[(2'-methylbiphenyl-4-yl)carbonyllpyrrolidin-3-one O-methyloxime

### Step 1: Preparation of (4R)-4-hydroxy-1-[(2'-methyl-1,1'-biphenyl-4-yl)-carbonyl]-L-proline (compound (IV) in scheme 4)

4-Hydroxy-*L*-proline (0.625wt) and water (3.3vol) were charged to a 20L flange flask. Triethylamine (2.42vol) was added to the contents dropwise such that the temperature was maintained in the range 10 to 20°C. Tetrahydrofuran (5.0vol) was added and the reaction mixture was cooled to 0 to 5°C. 2'-methyl-1,1'-biphenyl-4-carboxylic acid chloride, 1.0wt) and tetrahydrofuran (5.0vol) were charged to a separate flask, stirred for 5 to 10 minutes and then added to the reaction mixture ensuring that the temperature was maintained in the range 0 to 10°C. The reaction mixture was warmed to 15 to 25°C over 60-120 minutes and maintained at 15 to 25°C until reaction completion was noted by TLC analysis. The resultant is concentrated under vacuum at 35 to 40 °C, water (10.0vol) and ethyl acetate (5.0vol) were added to the residue and the contents stirred for 5 to 10 minutes. The layers were separated, the aqueous phase acidified to pH1 with aqueous hydrochloric acid (6M, approx. 3.0vol) and the resulting slurry cooled to and aged at 0 to 10°C for 25 to 40 minutes. The precipitate was collected by filtration, the isolated solid transferred to a suitable flange flask and slurried in warm (35 to 60°C) water (5.0vol) for 10 to 25 minutes. The solid was collected by filtration and the hot water slurry treatment was repeated as above. After the second slurry treatment the solid was azeotropically dried with toluene (2x 5.0vol) at 40 to 50 °C. Ethyl acetate (2.5vol) and heptanes (2.5vol) were added to the residue, the resulting slurry cooled to and aged 0 to 5°C for 30 to 40 minutes, filtered, the collected solids washed with pre-cooled (0 to 5°C) ethyl acetate : heptanes (1:1, 2.0vol) and dried under vacuum at 30 to 40°C to constant weight to give (4*R*)-4-hydroxy-1-[(2'-methyl-1,1'-biphenyl-4-yl)carbonyl]-*L*-proline as white solid. Yield: 85.9%.

### Step 2: Preparation of 1-[(2'-methyl-1,1'-biphenyl-4-yl)carbonyl]-4-oxo--L-proline (compound (V) in scheme 5)

(4*R*)-4-Hydroxy-1-[(2'-methyl-1,1'-biphenyl-4-yl)carbonyl]-*L*-proline (product of Step 1, 1.0wt) and dimethyl sulphoxide (2.5vol) were charged to a 20L flange flask. The contents were heated to 35 to 40 °C and maintained at this temperature until complete dissolution was achieved. The solution was cooled to 5 to 10 °C under a nitrogen atmosphere and triethylamine (3.0vol) was added such that the temperature was maintained in the range 5 to 20 °C. Pyridino-sulphur trioxide complex (1.47wt) and dimethyl sulphoxide (4.9vol) were charged to a separate flask, stirred for 5 to 10 minutes and then added to the reaction mixture such that the temperature was maintained in the range 15 to 25 °C. The reaction was stirred at 15 to 25 °C until reaction completion is noted by HPLC analysis (typically 1 to 3 hours). The vessel contents were cooled to 0 to 10 °C and quenched with aq. hydrochloric acid (3M, 8vol) maintaining the temperature below 30 °C. Tetrahydrofuran (5.0vol) and heptanes (1.0vol) were then added, the layers separated, the aqueous phase extracted with tetrahydrofuran (2x 5.0vol) and the combined organics washed with aq. hydrochloric acid (1M, 2x 2.0vol) and saturated brine solution (2x 2.0vol). The aqueous washes were combined and back-extracted with tetrahydrofuran (2x 1.0vol), the organics combined, dried over magnesium sulfate (3wt) and filtered. The filter-cake was washed with tetrahydrofuran (1.0vol) and the filtrates were concentrated under vacuum at 40 to 45 °C to give a pale brown foam. Ethyl acetate (10.0vol) was added to the residue, the contents stirred for 5 to 10 minutes and the solvent removed under vacuum at 40 to 45 °C. The residue was transferred to a flask, ethyl acetate (8.0vol) was added and the contents were heated to reflux. A slurry of activated carbon (0.14wt) in ethyl acetate (5.0vol) was added and reflux conditions reestablished and maintained for 20 to 30 minutes. The contents were cooled to 40 to 45 °C, filtered, the filter-cake was washed with ethyl acetate (2.5vol) and the filtrates concentrated to 2.5 to 3.0vol under vacuum at 40 to 45 °C. The slurry was diluted with ethyl acetate (0.5vol) and heated to reflux. Heptane (3.0vol) was added and the contents allowed to cool to 15 to 25 °C over 1 to 2 hours. The slurry was further cooled to at 0 to 5 °C for 2 to 3 hours, filtered and the filter-cake washed with ethyl acetate : heptane [(1:1), 1.0vol] pro-cooled to 0 to 5°C followed by heptane (5.0vol).

The isolated solid was dried under vacuum at 40 to 45°C to give 1-[(2'-methyl-1,1'-biphenyl-4-yl)carbonyl]-4-oxo-*L*-proline as off-white solid. Yield: 60.3%.

### Step 3: Preparation of 4-methoxyimino-1-[(2'-methyl-1,1'-biphenyl-4-yl)-carbonyl]-L-proline (compound (VII) in scheme 6)

1-[(2'-Methyl-1,1'-biphenyl-4-yl)carbonyl]-4-oxo-*L*-proline (of Step 2, 1.0wt), *O-*methyl-hydroxylamine hydrochloride (0.285wt) and dichloromethane (20vol) were charged to a 20L flange flask and cooled to 0 to 5 °C. Triethylamine (0.91vol) was charged to the flask such that the temperature was maintained in the range 0 to 10 °C, the reaction mixture was warmed to 15 to 25 °C and maintained within this temperature range for 16 to 20 hours. The reaction mixture was concentrated under vacuum at 40 to 45 °C, the residue dissolved in ethyl acetate (10.0vol) and washed with aq. hydrochloric acid (1M, 2x 5.0vol). The aqueous washes were combined and back extracted with ethyl acetate (5.0vol), the organic extracts combined and washed with saturated brine solution (10.0vol), dried over magnesium sulfate (0.5wt), filtered and the filter-cake washed with ethyl acetate (5.0vol). The filtrates were concentrated under vacuum at 40 to 45 °C to give 4-methoxyimino-1-[(2'-methyl-1,1' -biphenyl-4-yl)carbonyl]-*L*-proline in the expected *E*:*Z* mixture.

### Step 4a: Preparation of (3EZ, 5S)-1-[1-[(2'-Methylbiphenyl-4-yl)carbonyl]-5-(3-{2-[triethylsilyl)oxy]ethyl}-1,2,4-oxadiazol 5-yl)pyrrolidin-3-one O-methyloxime (compound (Ib) in scheme 7)

A solution of 4-methoxyimino-1-[(2'-methyl-1,1'-biphenyl-4-yl)carbonyl]-*L*-proline (80.0 g, 227.02 mmol, 1.00 eq) in tetrahydrofuran (1.00 L) was cooled to an internal temperature of 0 to 5 °C. Carbonyl diimidazole (38.65 g, 238.37 mmol, 1.05 eq) was added portionwise such that the internal temperature was maintained in the range 0 to 5 °C. The reaction mixture was warmed up and stirred at 20 to 25 °C until reaction completion (2 to 3h) was noted by HPLC analysis (quenching with 2.0 M ammonia in methanol). *N*-Hydroxy-3-triethylsilanyl-oxypropionamidine (81.25 g, 238.37 mmol, 1.05 eq., corrected for silanol content) as a solution in tetrahydrofuran (330 ml) was then added dropwise such that the internal temperature was kept between 20 to 25 °C and stirring was continued at 20 to 25 °C with reaction monitoring by HPLC analysis. Reaction completion was noted after 18 hours. The reaction mixture was concentrated under reduced pressure at 40 to 45 °C. Pyridine (500 ml) was added to the material and the resulting solution was heated to and maintained at 85 to 90 °C until HPLC analysis indicated complete cyclization (2 to 3 h). The reaction mixture was concentrated under reduced pressure at 40 to 45 °C, the dark oily residue treated with ethyl acetate (1.00 L) and washed with 25% aq. citric acid solution (3x 400 ml). The aqueous extracts were combined and back-extracted with ethyl acetate (250 ml), the combined organics washed with brine (1.00 L), dried over magnesium sulfate, filtered and the filter-cake washed with ethyl acetate. The combined filtrates were concentrated under reduced pressure at 40 to 45 °C to yield crude (3*EZ*, 5*S*)-1-[1-[(2'-methylbiphenyl-4-yl)carbonyl]-5-(3-{2-(triethylsilyl)oxy]ethyl}-1,2,4-oxadiazol-5-yl)pyrrolidin-3-one *O*-methyloxime as brown oil, which was used without any further purification (126.04 g, 104%).

### Step 4b: Preparation of (3Z,5S)-5-[3-(2-hydroxyethyl)-1,2,4-oxadiazol-5-yl]-1-[(2'-methylbiphenyl-4-yl)carbonyl]-3-pyrrolidinone O-methyloxime;

Crude (3*EZ*, 5*S*)-1-[1-[(2'-Methyl-1,1'-biphenyl-4-yl)carbonyl]-5-(3-{2-[triethylsilyl)oxy]ethyl}-1,2,4-oxadiazol-5-yl)pyrrolidin-3-one *O*-methyloxime (126.04 g) was combined with another batch of similar input (total amount: 257.8 g, 482.11 mmol, 1.0 eq.). Acetonitrile (1.29 L, 5.0vol) was added and the resulting solution was treated with a 5 % solution of trifluoroacetic acid in water (1.065 L). LCMS analysis indicated reaction completion after overnight stirring. Sodium hydrogen carbonate (48.5 g, 1.2 eq.) was added and the reaction mixture was stirred for 5 minutes. The product was extracted with ethyl acetate (3x 500 ml), the combined extracts were washed with semi-saturated brine (3x 300 ml), dried over magnesium sulfate, filtered and concentrated under reduced pressure at 40 to 45 °C to give a biphasic oily/solid residue. The residue was re-dissolved in acetonitrile (1.00 L), washed with heptane (3x 200 ml), separated and concentrated under reduced pressure at 40 to 45 °C to yield the title compound as brown oil (183.9 g, 90.7% th).

Purification of the *E*/*Z* product was performed with a column (Novasep, using silica, 40-63 microns; EtOAc/cyclohexane = 2:3, later on pure ethyl acetate), followed by an additional chromatography (Novasep, using silica, 15-25 microns; EtOAc/cyclohexane = 1:1). These two purifications allowed for removal of most by-products yielding a pale yellow oil. A third purification applying the same conditions as described for the second chromatography delivered pure Z isomer as colorless oil containing 5-10 % of the corresponding ketone. Dissolution in THF/DCM = 1:4 (total: 7vol), treatment with polymer-bound trisamine (1 g per 4.5 g of Z isomer) for 24 to 48 h, filtration and concentration under reduced pressure at 40 to 45 °C gave (3*Z*,5*S*)-5-[3-(2-hydroxyethyl)-1,2,4-oxadiazol-5-yl]-1-[(2'-methylbiphenyl-4-yl)carbonyl]-3-pyrrolidinone *O*-methyloxime as off-white solid (range of yield: 30-35 %).

### Example 3: (3EZ,5S)-1-([1,1'-biphenyl]-4-ylcarbonyl)-5-{5-[(dimethylamino)-methyl]-1,2,4-oxadiazol-3-yl}-3-pyrrolidinone O-methyloxime; (3Z,5S)-1-([1,1'-biphenyl]-4-ylcarbonyl)-5-{5-[(dimethylamino)-methyl]-1,2,4-oxadiazol-3-yl}-3-pyrrolidinone O-methyloxime; (3E,5S)-1-([1,1'-biphenyl]-4-ylcarbonyl)-5-{5-[(dimethylamino)-methyl]-1,2,4-oxadiazol-3-yl}-3-pyrrolidinone O-methyloxime

In this example, step 1, 2, and 3 are the same as in example 2.

### Step 4a: Preparation of (2S,4Z)-1-([1,1'-biphenyl]-4-ylcarbonyl)-4-(methoxy-imino)-2-pyrrolidinecarbonitrile (compound (VIIa) in scheme 7)

A 6L three-necked flask under a nitrogen atmosphere containing (2*S*)-1-(biphenyl-4-ylcarbonyl)-4-(methoxyimino)-pyrrolidine-2-carboxylic acid (151.95 g; 449.39 mmol; 1.00 eq.) in dry THF (2 500.00 ml) was cooled to -20°C prior to adding triethylamine (62.46 ml; 449.39 mmol; 1.00 eq.) (the temperature rose to -15°C) . The solution was stirred for 10 minutes and the temperature was brought to -35°C. Ethyl chloroformate (42.78 ml; 449.39 mmol; 1.00 cq.) was added to the solution over 10 minutes, maintaining the temperature at -35°C. The reaction mixture was stirred for 2h allowing the temperature to rise up to -20°C. An additional amount of 4 ml of ethyl chloroformate was added drop-wise over 5 minutes and the reaction mixture was stirred at -20°C for 30 minutes. An ammonia saturated THF solution was prepared by bubbling ammonia through 500ml of dry THF for 20 minutes at -60°C under a nitrogen atmosphere in a 2L three necked-flask. The ammonia solution was added to the reaction flask with a dropping funnel maintaining the temperature below -25°C. The solution was allowed to attain room temperature over 3h and the reaction mixture was stirred at overnight. The reaction mixture was cooled to 10°C and additional 250ml of an ammonia saturated THF solution were added drop-wise at -60°C within 10 minutes. Reaction was then stirred allowing temperature to warm to room temperature. Ammonia was directly bubbled in the reaction mixture at 15°C for 10 minutes after stirring for 3h. The reaction mixture was concentrated under vacuum to a volume of 1 L. The resulting slurry was filtered and the remaining residue was washed with 0.1N NaOH. The solid was rinsed with water and dried to give (2S,4Z)-1-([1,1'-biphenyl]-4-yl-carbonyl)-4-(methoxyimino)-2-pyrrolidinecarboxamide (102.10 g; 67.34%). A 3L three-necked flask containing (2S,4Z)-1-([1,1'-biphenyl]-4-yl-carbonyl)-4-(methoxyimino)-2-pyrrolidinecarboxamide (102.10 g; 302.63 mmol; 1.00 eq.) and toluene-4-sulfonyl chloride (86.54 g; 453.94 mmol; 1.50 eq.) in pyridine (1 500.00 ml) was stirred at 80°C overnight until completion. Volatile components were removed under vacuum and the residue was taken up in DCM (1L). The organic phase was washed with HCl 1N (2x 500ml) then with a saturated solution of NaHCO₃ (1 x500ml). The organic phase was dried over MgSO₄, filtered and concentrated to give a black residue (m=178g). This residue was taken up in DCM 350ml and the resulting suspension was filtered to give a cream powder. The filtrate was injected on a chromatographic column (Novasep) (dichloromethane) to be purified. Fractions of interest were combined and concentrated to give a brown residue, which was combined with the previously isolated solid (cream powder). The combined solids were diluted with methyl t-butyl ether (500ml), the suspension was filtered and rinsed with methyl t-butyl ether to give (2S,4Z)-1-([1,1'-biphenyl]-4-ylcarbonyl)-4-(methoxyimino)-2-pyrrolidinecarbonitrile (60.00 g; 62.08%).

### Step 4b: Preparation of (3EZ,5S)-1-([1,1'-biphenyl]-4-ylcarbonyl)-5-{5-[(dimethylamino)methyl]-1,2,4-axadiazol-3-yl}-3-pyrrolidinone O-methyloxime; (3Z,5S)-1-([1,1'-biphenyl]-4-ylcarbonyl)-5-{5-[(dimethylamino)methyl]-1,2,4-oxadiazol-3-yl}-3-pyrrolidinone O-methyloxime; (3E,5S)-1-([1,1'-biphenyl]-4-ylcarbonyl)-5-{5-[(dimethylamino)methyl]-1,2,4-oxadiazol-3-yl}-3-pyrrolidinone O-methyloxime

In a 2L three necked flask containing (2S,4Z)-1-([1,1'-biphenyl]-4-ylcarbonyl)-4-(methoxyimino)-2-pyrrolidinecarbonitrile (59.10 g; 185.06 mmol; 1.00 eq.) and hydroxylamine hydrochloride (15.43 g; 222.07 mmol; 1.20 eq.) in EtOH (1 200.00 ml) at room temperature, triethylamine (30.87 ml; 222.07 mmol; 1.20 eq.) was added drop-wise over 5 minutes. Then the reaction mixture was stirred at 80°C overnight to show completion. The temperature was allowed to cool to room temperature and the EtOH was removed under vacuum. Water (1L) was added and the suspension was filtered off. To remove any by-products, the solid was washed twice with acetonitrile (2x100m1) then with diethyl ether (1x100m1) to give a 75% pure product. After drying under vacuum at room temperature (2S,4Z)-1-(biphenyl-4-carbonyl)-N'-hydroxy-4-(methoxyimino)-pyrrolidine-2-carboximidamide (55.06 g; 84.43%) was obtained.
To a suspension of (2S,4Z)-1-(biphenyl-4-carbonyl)-N'-hydroxy-4-(methoxyimino)-pyrrolidine-2-carboximidamide (11.5 g; 32.63 mmol; 1.00 eq.), 4-dimethylaminopyridine (4.78 g; 39.16 mmol; 1.20 eq.), N,N-dimethylglycine (= R⁷-COOH; 4.04 g; 39.16 mmol; 1.20 eq.) in 1000ml of DCM/DMF(1:1), N-(3-dimethylaminopropyl)-N'-ethylcarbodiimide hydrochloride (6.88 g; 35.90 mmol; 1.10 eq.) was added. The resulting beige suspension was stirred at room temperature. Stirring was continued overnight. The solvent was removed under reduced pressure, the remaining oily brown residue was dissolved in dichlormethane, washed twice with 5 % citric acid (addition ofbrine was required to break the emulsion) and twice with sat. NaHCO₃, the organic layer was dried over MgSO₄ and concentrated under reduced pressure to give 12.45 g of a yellow-brownish solid. Said solid was split into three identical batches (~ 4.15 g), each batch was dissolved in 500 ml of pyridine and the resulting solutions were heated up to ET = 120°C o/n until completion. The batches were combined, the pyridine was removed under vacuum, the remaining residue was dissolved in DCM, washed twice with 5 % citric acid (phase separation was only possible after addition of brine due to formation of an emulsion), dried over MgSO₄ and evaporated under reduced pressure to give 12.9 g of a black oil. The crude product was pre-purified by plug filtration (silica; dichlormethane/MeOH = 95:5) to yield 10.67 g of a brown oil.
Purification of the E/Z product was performed with a column (using conventional silica; EtOAc/cyclohexane = 9:1). The first purification allowed to totally remove all by-products allowing for the isolation of the product as off-white solid (m = 6.73 g). A second purification applying the same conditions delivered pure Z isomer: (3Z,5S)-1-([1,1'-biphenyl]-4-ylcarbonyl)-5-{5-[(dimethylamino)methyl]-1,2,4-oxadiazol-3-yl}-3-pyrrolidinone *O*-methyloxime (4.937 g; 36 %).

### Example 3a: (3EZ,5S)-5-{5-[(dimethylamino)methyl]-1,2,4-oxadiazol-3-y-1}-1(2'-methylbiphenyl-4-yl)carbonyl]-pyrrolidin-3-one O-methyloxime; (3Z,5S)-5-{5-[(dimethylamino)methyl]-1,2,4-oxadiazol-3-yl}-1-[(2'-methylbiphenyl-4-yl)carbonyl]-pyrrolidin-3-one O-methyloxime: (3E,5S)-5-{5-[(dimethylamino)methyl]-1,2,4-oxadiazol-3-yl}-1-[(2'-methylbiphenyl-4-yl)carbonl]-pyrrolidin-3-one O-methyloxime

In this example, step 1, 2, and 3 are the same as in example 2a.

### Step 4a: Preparation of (2S,4EZ)-4-(methoxyimino)-1-[2'-methylbiphenyl-4-yl)carbonyl]pyrrolidine-2-carbonitrile (compound (VIIa) in scheme 7)

A 6L three-necked flask under a nitrogen atmosphere containing 4-methoxyimino-1-[(2'-methyl-1,1'-biphenyl-4-yl)carbonyl]-*L*-proline (150.00 g; 425.66 mmol; 1.00 eq.) in dry THF (2.5 L) was cooled to -25 °C prior to adding triethylamine (80.11 ml; 576.30 mmol; 1.63 eq.) (the temperature rose to -23°C). The solution was stirred for 10 minutes and the temperature was brought to -40 °C. Ethyl chloroformate (54.86 ml; 576.30 mmol; 1.63 eq.) was added to the solution over 30 minutes, maintaining the temperature below -35°C. The reaction mixture was stirred for 2.5 h allowing the temperature to rise up to -19 °C. An orange suspension was obtained. An ammonia saturated THF solution was prepared by bubbling ammonia through 500 ml of dry THF for 20 minutes at -40 °C under a nitrogen atmosphere in a 1L three necked-flask. The ammonia solution (400 ml) was added to the reaction flask with a dropping funnel maintaining the temperature below -25 °C. The obtained solution was allowed to attain -20°C within 1h, after which the reaction was found to be complete and further to warm up to room temperature overnight. The reaction mixture was concentrated under vacuum to a volume of 200 ml and the remaining residue was diluted with 600 ml of MTBE. The resulting suspension was filtered, the filter cake was rinsed with MTBE (2x 200 ml), the collected filtrates were further diluted with ethyl acetate (400 ml) and washed with water (2x 500 ml). The aqueous phase was back-extracted with ethyl acetate (300 ml), the combined organic phases were dried over magnesium sulfate, filtered and concentrated under reduced pressure to give (2*S*,4*EZ*)-4-(methoxyimino)-1-[(2'-methylbiphenyl-4-yl)carbonyl]-L-prolinamide (163.64; 109.4%th). The product was further used without purification. A 3L three-necked flask containing (2*S*,4*EZ*)-4-(methoxyimino)-1-[(2'-methylbiphenyl-4-yl)carbonyl]-L-prolinamide (149.56 g; 425.61 mmol; 1.00 eq., the calculation was based on 100 % yield of the previous step) and toluene-4-sulfonyl chloride (121.71 g; 638.41 mmol; 1.50 eq.) in pyridine (1.5 L) was stirred at 80 °C until completion (4.5 h). Volatile components were removed under vacuum at 40 to 45 °C and the residue was taken up in DCM (1L). The organic phase was washed with HCl 1N (2x 500ml) then with a saturated solution of NaHCO₃ (1x 500ml). The organic phase was dried over magnesium sulfate, filtered and concentrated to give a black residue. This residue was taken up in DCM (350 ml) and injected on a chromatographic column (Novasep) (dichloromethane) to be purified. Fractions of interest were combined and concentrated to give a brown residue, which was used without any further purification: Yield: (2*S*,4*EZ*)-4-(methoxyimino)-1-[(2'-methylbiphenyl-4-yl)carbonyl]pyrrolidine-2-carbonitrile (136.80 g; 65.83%).

### Step 4b: Preparation of (3EZ,5S)-5- {5-[(dimethylamino)methyl]-1,2,4-oxadiazol-3-yl}-1-[(2'-methylbiphenyl-4-yl)carbonyl]pyrrolidin-3-one O-methyloxime; (3Z,5,5')-5-{5-[(dimethylamino)methyl]-1,2,4-oxadiazol-3-yl}-1-[(2'-methylbiphenyl-4.-yl)carbonyl]pyrrolidin-3-one O-methyloxime; (3E,5S)-5-{5-[(dimethylamino)methyl]-1,2,4-oxadiazol-3-yl}-1-[(2'-methylbiphenyl-4-yl)carbonyl]pyrrolidin-3-one O-methyloxime

To a 2L three necked flask containing (2S,4EZ)-4-(methoxyimino)-1-[2'-methylbiphenyl-4-yl)carbonyl]pyrrolidine-2-carbonitrile (136.38 g; 278.70 mmol; 1.00 eq.) and hydroxylamine hydrochloride (27.11 g; 390.18 mmol; 1.40 eq.) in ethanol (1.5 L) at room temperature, triethylamine (54.23 ml; 390.18 mmol; 1.40 eq.) was added dropwise over 5 minutes. Then the reaction mixture was stirred at 80 °C overnight to show completion. The temperature was allowed to cool to room temperature and the ethanol was removed under vacuum. Water (1 L) was added and the suspension was filtered. The remaining solid was washed twice with acetonitrile (2x 150ml) and dried under vacuum at room temperature to yield (2S,4*EZ*)-N'-hydroxy-4-(methoxyimino)-1-[(2'-methylbiphenyl-4-yl)carbonyl]pyrrolidine-2-carboximidamide (52.00 g; 50.92%).
To a suspension of (2*S*,4*EZ*)- N'-hydroxy-4-(methoxyimino)-1-[(2'-methylbiphenyl-4-yl)carbonyl]pyrrolidine-2-carboximidamide (19.00 g; 51.85 mmol; 1.00 eq.), 4-dimethylamino-pyridine (7.60 g; 62.22 mmol; 1.20 eq.), N,N-dimethylglycine (= R⁷-COOH; 6.42 g; 62.22 mmol; 1.20 eq.) in 1.8 L of DCM/DMF(1:1), N-(3-dimethylaminopropyl)-N'-ethylcarbodiimide hydrochloride (10.93 g; 57.04 mmol; 1.10 eq.) were added. The resulting suspension was stirred at room temperature overnight upon which a solution was formed. The solvent was removed under reduced pressure, the remaining residue was dissolved in dichloromethane, washed twice with 5 % citric acid and twice with sat. NaHCO₃, the organic layer was dried over magnesium sulfate and concentrated under reduced pressure to give 24.57 g of a brownish amorphous solid. Said solid was split into five identical batches (~4.91 g), each batch was dissolved in 200 ml of pyridine and the resulting solutions were heated up to ET = 120°C o/n until completion. The batches were combined, the pyridine was removed under vacuum and the remaining residue was pre-purified by chromatography (Novasep, 100 % ethyl acetate) to give a brown oil (m = 10.28 g). Purification by flash chromatography applying the same conditions yielded a yellow oil (m = 2.62 g), which was repeatedly purified under the same conditions to give the title compound as pure Z isomer: (3Z,5S)-5-{5-[(dimethylamino)methyl]-1,2,4-oxadiaxol-3-yl} -1-[(2'-methylbiphenyl-4-yl)carbonyl]pyrrolidin-3-one *O-*methyloxime (3.54 g; 15 %).

### Example 4: (3Z/E, 5S)-1-(biphenyl-4-yl carbonyl)-5-hydroxymethyl) pyrrolidine-3-one-O-methyloxime

In this example, step 1, 2, and 3 are the same as in example 1.

### Step 4: (4Z/E, 2S) Methyl-1-(biphenyl-4-yl carbonyl)-4-methoxyimino) pyrrolidine-2-carboxylate) (esterification in scheme 7)

4-Methoxyimino-1-[(2'-methyl-1,1'-biphenyl-4-yl)-carbonyl]-*L*-proline (1wt), acetone (10 vol) and potassium carbonate (1 wt) were charged to a suitably sized flask under nitrogen. The contents were cooled to a temperature between 0 and 10°C and dimethyl sulphate was added, maintaining the temperature below 10°C. The reaction was warmed to a temperature between 16 and 25°C and maintained in this temperature range until judged complete (expected time: 1 to 2 hours). The contents were concentrated under vacuum at 40 to 45°C. Ethyl acetate (8 vol) and water (8 vol) were charged to the residue and the layers were separated. The organic phase was washed with saturated brine solution (8 vol) and then dried over sodium sulphate (2 wt). The contents were filtered, the filter-cake was washed with ethyl acetate (1 vol) and the filtrates were concentrated under vacuum at 40 to 45°C. The residue was dissolved in dichloromethane (1 vol) and the resultant solution was split in half for chromatography. Each solution was purified by dry flash chromatography using silica (1.8 wt) and eluting with 25%v/v ethyl acetate in heptanes (12 vol) followed by 50%v/v ethyl acetate/heptanes (12 vol) to remove minor impurities. The fractions containing product from the 2 columns were combined and concentrated under vacuum at 40 to 45°C. The residue was dissolved in THF (2.5 vol) and reconcentrated under vacuum at 40 to 45°C to give the desired product (80 to 100%, 83 to 104%w/w).

### Step 5: Preparation of (3Z/E, 5S)-1-(biphenyl-4-yl carbonyl)-5-hydroxymethyl) pyrrolidine-3-one-O-methyloxime (reduction to compound (Id) in scheme 7)

(4*Z*/*E*, 2*S*) Methyl-1-(biphenyl-4-yl carbonyl)-4-methoxyimino) pyrrolidine-2-carboxylate (1wt), THF (4.7 vol) and methanol (4.7 vol) were charged to a suitably sized flask. The solution was cooled to a temperature between 0 and 10°C under nitrogen and lithium borohydride (0.1 wt) was added portionwise, maintaining the temperature below 20°C. The reaction was stirred at 16 to 25°C until judged complete by TLC (expected time: 2 to 3 hours). The reaction was quenched by the addition of water (0.8 vol) and concentrated under vacuum at 40 to 45°C. Ethyl acetate (10 vol) and water (5 vol) were added to the residue and the layers were separated. The aqueous phase was back extracted with ethyl acetate (2 vol). The organic phases were combined and washed with 1M HCI (5 vol), saturated NaHCO₃ (5 vol) and saturated brine solution (5 vol). The organic solution was dried over magnesium sulphate (2 wt). The contents were filtered and the filtrate was concentrated under vacuum at 40 to 45°C to give the desired product (80 to 100%, 74 to 92%w/w). The crude product was subsequently purified.

## Claims

1. A method of preparing a compound according to formula (I): 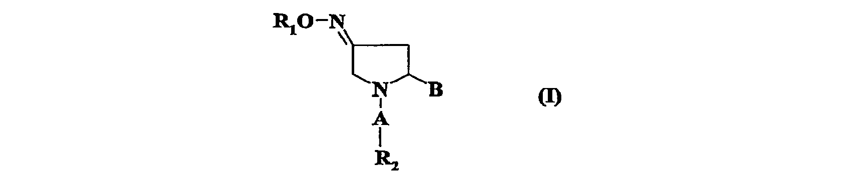 wherein
A is a carbonyl group -(C=O)-;
B is selected from the group consisting of an oxadiazole ring, an amido group of the formulae -(C=O)-NR₃R₄, and -(CH₂)n-X-R₈;
wherein the oxadiazole ring is any of the formulae: 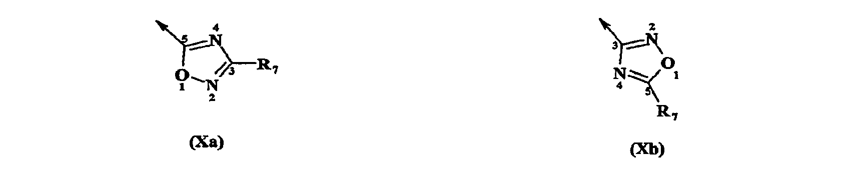 R₁ is H or a C₁-C₆-alkyl;
R₂ is selected from the group consisting of aryl, heteroaryl and saturated or unsaturated 3-8-membered cycloalkyl;
R₃ and R₄ are independently selected from the group consisting of hydrogen, C₁-C₆ alkyl, C₂-C₆ alkenyl, C₂-C₆ alkynyl, alkoxy, sulfanyl, acyl, alkoxycarbonyl, aminocarbonyl, saturated or unsaturated 3-8-membered cycloalkyl which may contain 1 to 3 heteroatoms selected ofN, O, S, aryl, heteroaryl, C₁-C₆-alkyl aryl and C₁-C₆-alkyl heteroaryl;
X is O or NR₉;
R₈ is selected from the group consisting of hydrogen, C₁-C₆-alkyl, C₁-C₆-alkyl aryl, heteroaryl, C₁-C₆-alkyl heteroaryl, C₂-C₆-alkenyl, C₂-C₆-alkenyl aryl, C₂-C₆-alkenyl heteroaryl, C₂-C₆-alkynyl, C₂-C₆-alkynyl aryl, C₂-C₆-alkynyl heteroaryl, C₃-C₈-cycloalkyl, heterocycloalkyl, C₁-C₆-alkyl cycloalkyl, C₁-C₆-alkyl heterocycloalkyl, C₁-C₆-alkyl carboxy, acyl, C₁-C₆-alkyl acyl, C₁-C₆-alkyl acyloxy, C₁-C₆-alkyl alkoxy, alkoxycarbonyl, C₁-C₆-alkyl alkoxycarbonyl, aminocarbonyl, C₁-C₆-alkyl aminocarbonyl, C₁-C₆-alkyl acylamino, C₁-C₆-alkyl ureido, amino, C₁-C₆-alkyl amino, sulfonyloxy, C₁-C₆-alkyl sulfonyloxy, sulfonyl, C₁-C₆-alkyl sulfonyl, sulfinyl, C₁-C₆-alkyl sulfinyl, C₁-C₆-alkyl sulfanyl and C₁-C₆-alkyl sulfonylamino;
R₇ is selected from the group consisting of hydrogen, sulfonyl, amino, C₁-C₆-alkyl, C₂-C₆-alkenyl, C₂-C₆-alkyoyl, wherein said alkyl, alkenyl, alkynyl chains are optionally interrupted by a heteroatom selected from N, O or S, aryl, heteroaryl, saturated or unsaturated 3-8-membered cycloalkyl, heterocycloalkyl, wherein said cycloalkyl, heterocycloalkyl, aryl or heteroaryl groups are optionally fused with 1-2 further cycloalkyl, heterocycloalkyl, aryl or heteroaryl group, an acyl moiety, C₁-C₆-alkyl aryl, C₁-C₆-alkyl heteroaryl, C₁-C₆-alkenyl aryl, C₁-C₆-alkenyl heteroaryl, C₁-C₆-alkynyl aryl, C₁-C₆-alkynyl heteroaryl, C₁-C₆-alkyl cycloalkyl, C₁-C₆-alkyl heterocycloalkyl, C₁-C₆-alkenyl cycloalkyl, C₁-C₆-alkenyl heterocycloalkyl, C₁-C₆-alkynyl cycloalkyl, C₁-C₆-alkynyl heterocycloalkyl, alkoxycarbonyl, aminocarbonyl, C₁-C₆-alkyl carboxy, C₁-C₆-alkyl acyl, C₁-C₆-alkyl acyloxy, C₁-C₆-alkyl alkoxy, C₁-C₆-alkyl alkoxy-carbonyl, C₁-C₆-alkyl aminocarbonyl, C₁-C₆-alkyl acylamino, C₁-C₆-alkyl urcido, C₁-C₆-alkyl amino, C₁-C₆-alkyl ammonium, C₁-C₆-alkyl sulfonyloxy, C₁-C₆-alkyl sulfonyl, C₁-C₆-alkyl sulfinyl, C₁-C₆-alkyl sulfanyl, C₁-C₆-alkyl sulfonylamino, C₁-C₆-alkyl aminosulfonyl, hydroxy, halogen and cyano;
R₉ is selected from the group consisting of hydrogen, C₁-C₆-alkyl, C₁-C₆-alkyl aryl, C₁-C₆-alkyl heteroaryl, aryl and heteroaryl;
R₈ and R₉ can form together with the N atom to which they are linked to, a 5-8 membered saturated or unsaturated heterocycloalkyl ring; and
n is an integer from 1 to 3;
said method comprises the following steps :
**Step 1 :** transformation of the pyrrolidine of formula (II) into an acyl derivative of formula (IV) using an acylating agent (III): 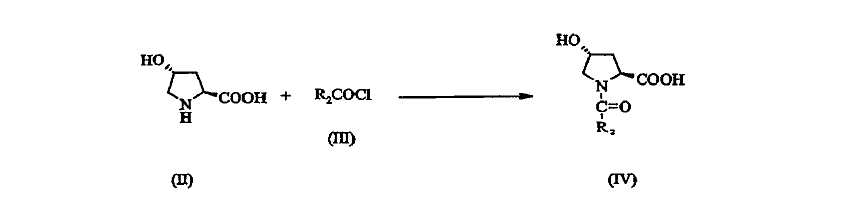
**Step 2 :** Oxidation of the acyl derivative (IV), with a oxidizing agent, obtaining a pyrrolidone of formula (V) : 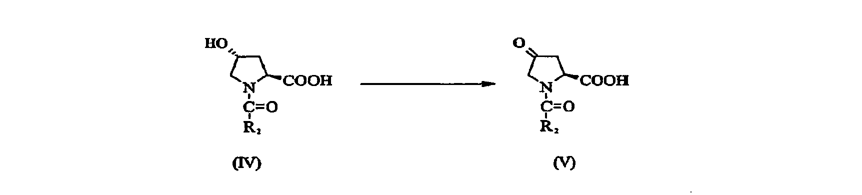
**Step 3 :** Transformation of the pyrrolidone of formula (V) into compound (VII) using a suitable alkoxylamine, aryloxylamine or hydroxylamine of general formula (VI) : 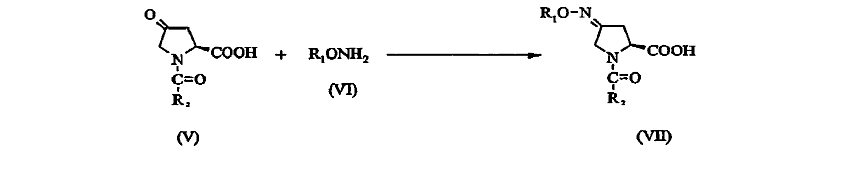
**Step 4 :** Transformation of the compound (VII) with an amine of general formula (VIII) or an N-hydroxyamidine of general formula (IX) thus yielding compounds (Ia) and (Ib), or transforming compound (VII) first into a nitrile (VIIa), which is then transformed into the hydroxyamidine (VIIb) that is then reacted with a carboxylic acid
R⁷-COOR to yield compound (Ic), or first esterifying and than reducing compound (VII) using a suitable esterification or reducing agent, respectively, thus yielding compound (Id):

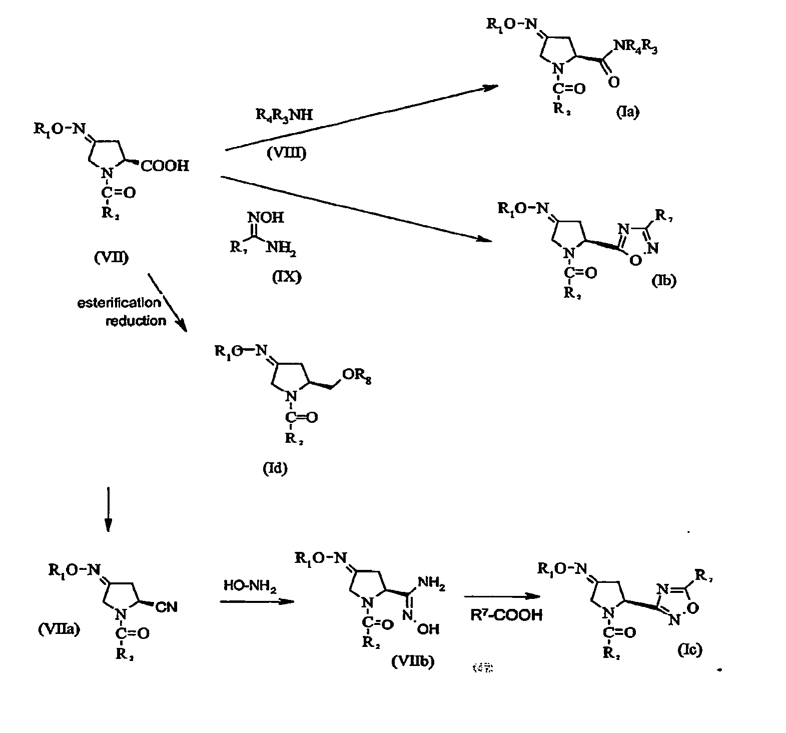

2. The method of preparing a compound according to formula (I) according to claim 1: 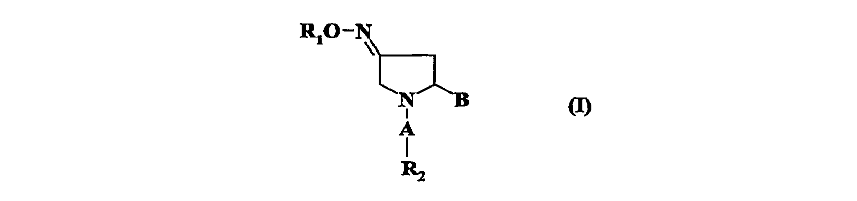 wherein
A is a carbonyl group -(C=O)-;
B is either an amido group of formula -(C=O)-NR₃R₄. or an oxadiazole ring of any of the formulae: 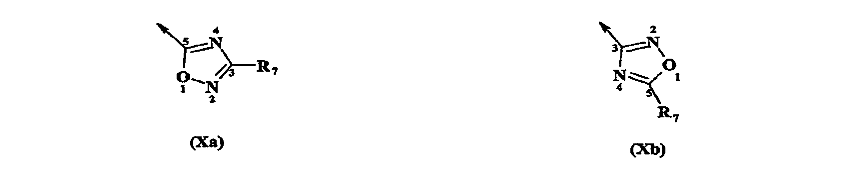 R₇ is selected from the group consisting of hydrogen, sulfonyl, amino, C₁-C₆-alkyl, C₂-C₆-alkenyl, C₂-C₆-alkynyl, wherein said alkyl, alkenyl, alkynyl chains are optionally interrupted by a heteroatom selected from N, O or S, aryl, heteroaryl, saturated or unsaturated 3-8-membered cycloalkyl, heterocycloalkyl, wherein said cycloalkyl, heterocycloalkyl, aryl or heteroaryl groups are optionally fused with 1-2 further cycloalkyl, heterocycloalkyl, aryl or heteroaryl group, an acyl moiety, C₁-C₆-alkyl aryl, C₁-C₆-alkyl heteroaryl, C₁-C₆-alkenyl aryl, C₁-C₆-alkenyl heteroaryl, C₁-C₆-alkynyl aryl, C₁-C₆-alkynyl heteroaryl, C₁-C₆-alkyl cycloalkyl, C₁-C₆-alkyl heterocycloalkyl, C₁-C₆-alkenyl cycloalkyl, C₁-C₆-alkenyl heterocycloalkyl, C₁-C₆-alkynyl cycloalkyl, C₁-C₆-alkynyl heterocycloalkyl alkoxycarbonyl, aminocarbonyl , C₁-C₆-alkyl carboxy, C₁-C₆-alkyl acyl, C₁-C₆-alkyl acyloxy, C₁-C₆-alkyl alkoxy, C₁-C₆-alkyl alkoxy-carbonyl, C₁-C₆-alkyl aminocarbonyl, C₁-C₆-alkyl acylamino, C₁-C₆-alkyl ureido, C₁-C₆-alkyl amino, C₁-C₆-alkyl ammonium, C₁-C₆-alkyl sulfonyloxy, C₁-C₆-alkyl sulfonyl, C₁-C₆-alkyl sulfinyl, C₁-C₆-alkyl sulfanyl, C₁-C₆-alkyl sulfonylamino, C₁-C₆-alkyl aminosulfonyl, hydroxy, halogen and cyano;
R₁ is H or a C₁-C₆-alkyl;
R₂ is selected from the group consisting of aryl, heteroaryl and saturated or unsaturated 3-8-membered cycloalkyl;
R₃ and R₄ are independently selected from the group consisting of hydrogen, C₁-C₆ alkyl, C₂-C₆ alkenyl, C₂-C₆ alkynyl, alkoxy, sulfanyl, acyl, alkoxycarbonyl, aminocarbonyl, saturated or unsaturated 3-8-membered cycloalkyl which may contain 1 to 3 heteroatoms selected ofN, O, S, aryl, heteroaryl, C₁-C₆-alkyl aryl and C₁-C₆-alkyl heteroaryl;
said method comprises the following steps :
**Step 1:** transformation of the pyrrolidine of formula (II) into an acyl derivative of formula (IV) using an acylating agent (III) : 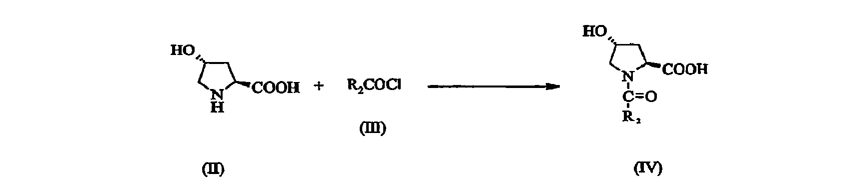
**Step 2:** Oxidation of the acyl derivative (IV), with a oxidizing agent, obtaining a pyrrolidone of formula (V): 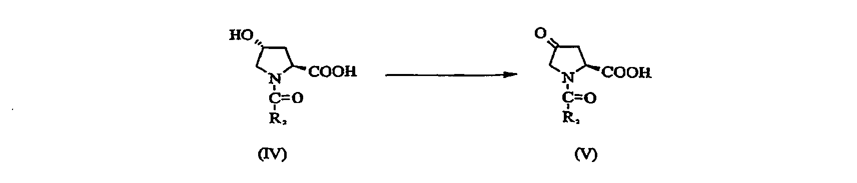
**Step 3:** Transformation of the pyrrolidone of formula (V) into compound (VII) using a suitable alkoxylamine, aryloxylamine or hydroxylamine of general formula (VI): 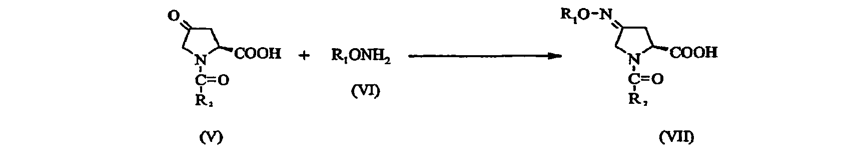
Step 4 : Transformation of the compound (VII) with an amine of general formula (VIII) or an N-hydroxyamidine of general formula (IX) thus yielding compounds (Ia) and (Ib), or transforming compound (VII) first into a nitrile (VIIa), which is then transformed into the hydroxyamidine (VIIb) that is then reacted with a carboxylic acid R⁷-COOR to yield compound (Ic).:

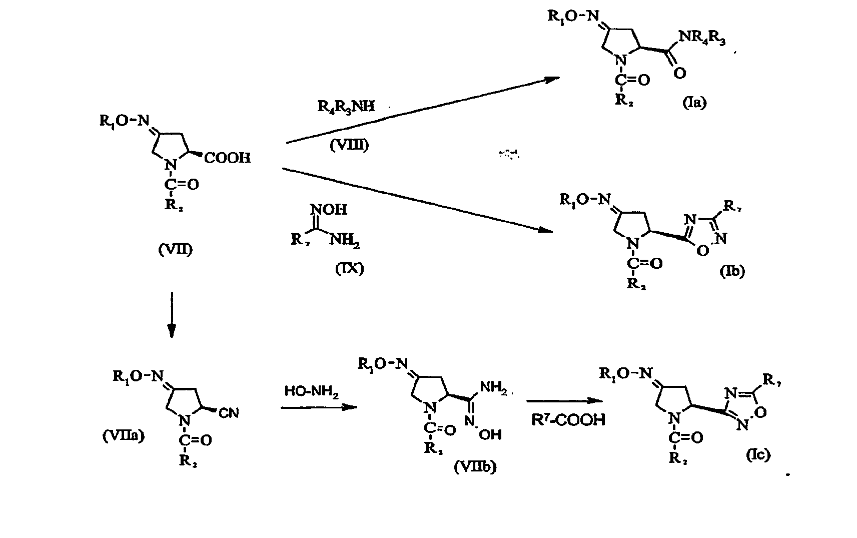

3. The method according to claim 1 or 2, wherein the acyl chloride of step 1 is 1'1-biphenyl-4-carbonyl chloride or 2'-methyl-1'1-biphenyl-4-carbonyl chloride.

4. The method according to any of claims 1 to 3, wherein the *oxidizing* agent of Step 2 is pyridine-sulfurtrioxide complex (Py-SO₃) in combination with DMSO.

5. The method according to any of claims 2 to 4, wherein the reaction is performed in presence of triethylamine.

6. The method according to any of claims 1 to 5, wherein the alkoxylamine used in step 3 is O-methylhydroxylamine hydrochloride.

7. The method according to any of claims 1 to 6, wherein R₁ is a methyl group, R₂ is a biphenyl.

8. The method according to any of claims 1 to 7, wherein B is an amido group of the formula -(C=O)NHR₅, with R₅ being an C₁-C₆-alkyl aryl group.

9. The method according to claim 8, wherein R₅ is a phenylethyl group, which is substituted with an amino or hydroxy group.

10. The method according to any of claims 1 to 7, wherein B is a 1,2,4 oxadiazole substituent 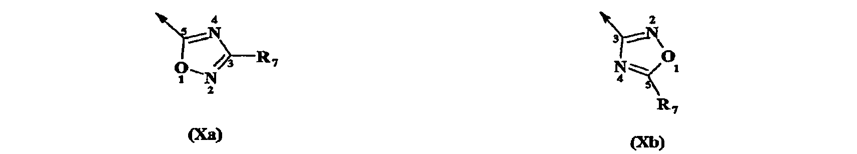 with R₇ being a C₁-C₆-alkyl or a cycloalkyl optionally containing one or 2 hetereroatoms.

11. The method according to any of claims 1, 3, 4, or 6 to 7, wherein B is -(CH₂)n-X-R₈, with X being O, R₈ being hydrogen; and n being 1.

12. The method according to any of claims 1 to 11, wherein the compound is selected from the group consisting of:
(2*S*,4*E* and 4*Z*)-*N*-[(2*S*)-2-hydroxy-2-phenylethyl]-4-(methoxyimino)-1-[(2'-methyl[1,1'-biphenyl]-4-yl)carbonyl]-2-pyrrolidine carboxamide,
(3*E*,5*S*)-1-([1,1'-biphenyl]-4-ylcarbonyl)-5-[3-(2-hydroxyethyl)-1,2,4-oxadiazol-5-yl]-3-pyrrolidinone *O*-methyloxime,
(3*Z*,5*S*)-1-([1,1'-biphenyl]-4-ylcarbonyl)-5-[3-(2-hydroxyethyl)-1,2,4-oxadiazol-5-yl]-3-pyrrolidinone *O*-methyloxime,
(3*E*,5*S*)-5-[3-(2-hydroxyethyl)-1,2,4-oxadiazol-5-yl]-1-[(2'-methylbiphenyl-4-yl)carbonyl]pyrrolidin-3-one *O*-methyloxime,
(3*Z*,*5S*)-5-[3-(2-hydroxyethyl)-1,2,4-oxadiazol-5-yl]-1-[(2'-methylbiphenyl-4-yl)carbonyl]pyrrolidin-3-one *O*-methyloxime,
(3*EZ*,*5S*)-1-([1,1'-biphenyl]-4-ylcarbonyl)-5-{5-[(dimethylamino)-methyl]-1,2,4-oxadiazol-3-yl}-3-pyrrolidinone *O*-methyloxime,
(3*Z*,*5S*)-1-([1,1'-biphenyl]-4-ylcarbonyl)-5-{5-[(dimethylamino)-methyl]-1,2,4-oxadiazol-3-yl}-3-pyrrolidinone *O*-methyloxime,
(3*E*,*5S*)-1-([1,1'-biphenyl]-4-ylcarbonyl)-5-{5-[(dimethylamino)-methyl]-1,2,4-oxadiazol-3-yl}-3-pyrrolidinone *O*-methyloxime,
(3*EZ*,*5S*)-5-{5-[(dimethylamino)methyl]-1,2,4-oxadiazol-3-yl}-1-[(2'-methylbiphenyl-4-yl)carbonyl]-pyrrolidin-3-one *O*-methyloxime,
(3*Z*,5*S*)-5-{5-[(dimethylamino)methyl]-1,2,4-oxadiazol-3-yl}-1-[(2'-methylbiphenyl-4-yl)carbonyl]-pyrrolidin-3-one *O*-methyloxime,
(3E,SS)- 5-{5-[(dimethylamino)methyl]-1,2,4-oxadiazol-3-yl}-1-[(2'-methylbiphenyl-4-yl)carbonyl]-pyrrolidin-3-one *O*-methyloxime, and
(3Z/E, 5S)-1-(biphenyl-4-yl carbonyl)-5-hydroxymethyl) pyrrolidine-3-one-O-methyloxime.

## Patentansprüche

1. Verfahren zur Herstellung einer Verbindung der Formel (I): 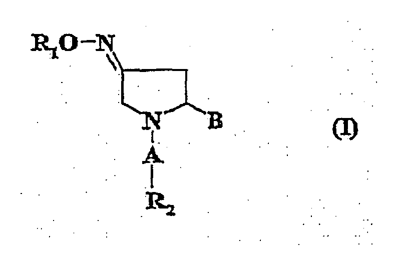 worin
A eine Carbonylgruppe -(C=O)- ist;
B ausgewählt ist aus der Gruppe, bestehend aus einem Oxadiazolring, einer Amidogruppe der Formel -(C=O)-NR₃R₄ und - (CH₂)ₙ-X-R₈;
wobei der Oxadiazolring einer der Formeln: 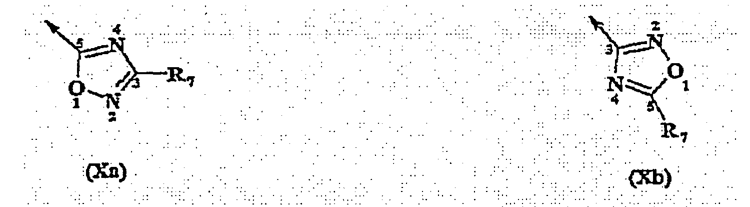 ist;
R₁ Wasserstoff oder ein C₁-C₆-Alkyl ist;
R₂ ausgewählt ist aus der Gruppe, bestehend aus Aryl, Heteroaryl und gesättigtem oder ungesättigtem 3-8-gliedrigem Cycloalkyl;
R₃ und R₄ unabhängig voneinander ausgewählt sind aus der Gruppe, bestehend aus Wasserstoff, C₁-C₆-Alkyl, C₂-C₆-Alkenyl, C₂-C₆-Alkinyl, Alkoxy, Sulfanyl, Acyl, Alkoxycarbonyl, Aminocarbonyl, gesättigtem oder ungesättigtem 3-8-gliedrigem Cycloalkyl mit gegebenenfalls 1-3 Heteroatomen, ausgewählt aus N, O, S, Aryl, Heteroaryl, C₁-C₆-Alkylaryl und C₁-C₆-Alkylheteroaryl;
X O oder NR₉ ist;
R₈ ausgewählt ist aus der Gruppe, bestehend aus Wasserstoff, C₁-C₆-Alkyl, C₁-C₆-Alkylaryl, Heteroaryl, C₁-C₆-Alkylheteroaryl, C₂-C₆-Alkenyl, C₂-C₆-Alkenylaryl, C₂-C₆-Alkenylheteroaryl, C₂-C₆-Alkinyl, C₂-C₆-Alkinylaryl, C₂-C₆-Alkinylheteroaryl, C₃-C₈-Cycloalkyl, Heterocycloalkyl, C₁-C₆-Alkylcycloalkyl, C₁-C₆-Alkylheterocycloalkyl, C₁-C₆-Alkylcarboxy, Acyl, C₁-C₆-Alkylacyl, C₁-C₆-Alkylacyloxy, C₁-C₆-Alkylalkoxy, Alkoxycarbonyl, C₁-C₆-Alkylalkoxycarbonyl, Aminocarbonyl, C₁-C₆-Alkylaminocarbonyl, C₁-C₆-Alkylacylamino, C₁-C₆-Alkylureido, Amino, C₁-C₆-Alkylamino, Sulfonyloxy, C₁-C₆-Alkylsulfonyloxy, Sulfonyl, C₁-C₆-Alkylsulfonyl, Sulfinyl, C₁-C₆-Alkylsulfinyl, C₁-C₆-Alkylsulfanyl und C₁-C₆-Alkylsulfonylamino;
R₇ ausgewählt ist aus der Gruppe, bestehend aus Wasserstoff, Sulfonyl, Amino, C₁-C₆-Alkyl, C₂-C₆-Alkenyl, C₂-C₆-Alkinyl, wobei die Alkyl-, Alkenyl-, Alkinylketten gegebenenfalls unterbrochen sind durch ein Heteroatom, ausgewählt aus N, O oder S, Aryl, Heteroaryl, gesättigtem oder ungesättigtem 3-8-gliedrigem Cycloalkyl, Heterocycloalkyl, wobei die Cycloalkyl-, Heterocycloalkyl-, Aryl- oder Heteroarylgruppen gegebenenfalls anneliert mit 1-2 weiteren Cycloalkyl-, Heterocycloalkyl-, Aryl- oder Heteroarylgruppen, einem Acylrest, C₁-C₆-Alkylaryl, C₁-C₆-Alkylheteroaryl, C₁-C₆-Alkenylaryl, C₁-C₆-Alkenylheteroaryl, C₁-C₆-Alkinylaryl, C₁-C₆-Alkinylheteroaryl, C₁-C₆-Alkylcycloalkyl, C₁-C₆-Alkylheterocycloalkyl, C₁-C₆-Alkenylcycloalkyl, C₁-C₆-Alkenylheterocycloalkyl, C₁-C₆-Alkinylcycloalkyl, C₁-C₆-Alkinylheterocycloalkyl, Alkoxycarbonyl, Aminocarbonyl, C₁-C₆-Alkylcarboxy, C₁-C₆-Alkylacyl, C₁-C₆-Alkylacyloxy, C₁-C₆-Alkylalkoxy, C₁-C₆-Alkylalkoxycarbonyl, C₁-C₆-Alkylaminocarbonyl, C₁-C₆-Alkylacylamino, C₁-C₆-Alkylureido, C₁-C₆-Alkylamino, C₁-C₆-Alkylammonium, C₁-C₆-Alkylsulfonyloxy, C₁-C₆-Alkylsulfonyl, C₁-C₆-Alkylsulfinyl, C₁-C₆-Sulfanyl, C₁-C₆-Alkylsulfonylamino, C₁-C₆-Alkylaminosulfonyl, Hydroxy, Halogen und Cyano;
R₉ ausgewählt ist aus der Gruppe, bestehend aus Wasserstoff, C₁-C₆-Alkyl, C₁-C₆-Alkylaryl, C₁-C₆-Alkylheteroaryl, Aryl und Heteroaryl;
R₈ und R₉ zusammen mit dem N-Atom, an das sie gebunden sind, einen 5-8-gliedrigen gesättigten oder ungesättigten Heterocycloalkylring bilden können; und
n eine ganze Zahl von 1 bis 3 ist;
wobei das Verfahren die folgenden Schritte umfasst:
**Schritt 1:** Überführung des Pyrrolidins der Formel (II) in ein Acylderivat der Formel (IV) unter Verwendung eines Acylierungsmittels (III) : 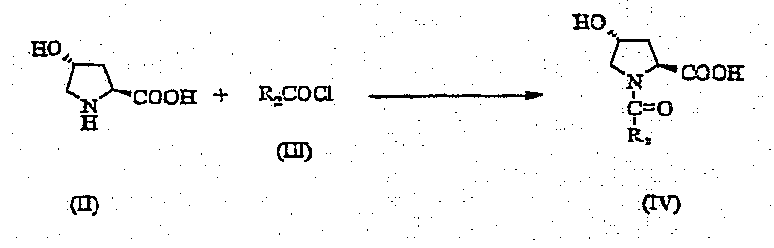
**Schritt 2:** Oxidation des Acylderivats (IV) mit einem Oxidationsmittel unter Erhalt eines Pyrrolidons der Formel (V) 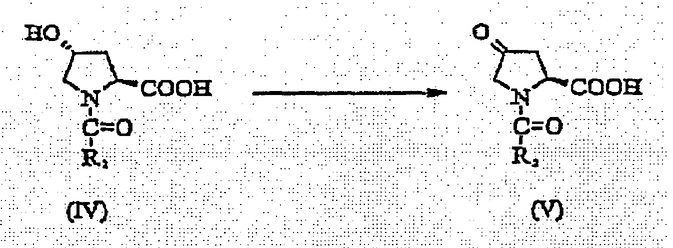
**Schritt 3:** Überführung des Pyrrolidons der Formel (V) in Verbindung (VII) unter Verwendung eines geeigneten Alkoxylamins, Aryloxylamins oder Hydroxylamins der allgemeinen Formel (VI): 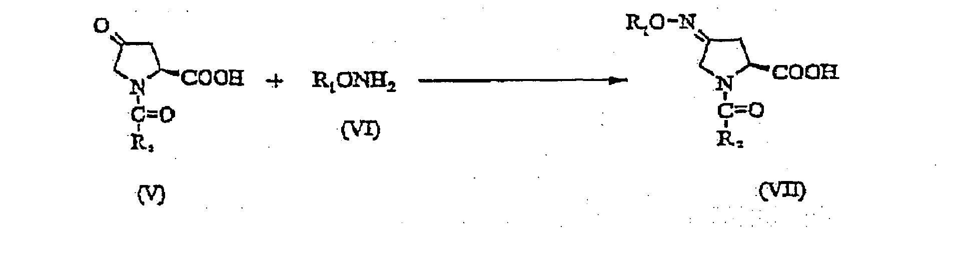
**Schritt 4:** Überführung der Verbindung (VII) mit einem Amin der allgemeinen Formel (VIII) oder einem N-Hydroxyamidin der allgemeinen Formel (IX), wobei Verbindungen (Ia) und (Ib) erhalten werden, oder Überführung der Verbindung (VII) zunächst in ein Nitril (VIIa), welches anschließend überführt wird in das Hydroxyamidin (VIIb), welches anschließend umgesetzt wird mit einer Carbonsäure R⁷-COOH, um Verbindung (Ic) zu erhalten, oder es erfolgt zunächst eine Veresterung und anschließend eine Reduktion der Verbindung (VII) unter Verwendung eines geeigneten Veresterungs- oder Reduktionsmittels, wobei Verbindung (Id) erhalten wird:

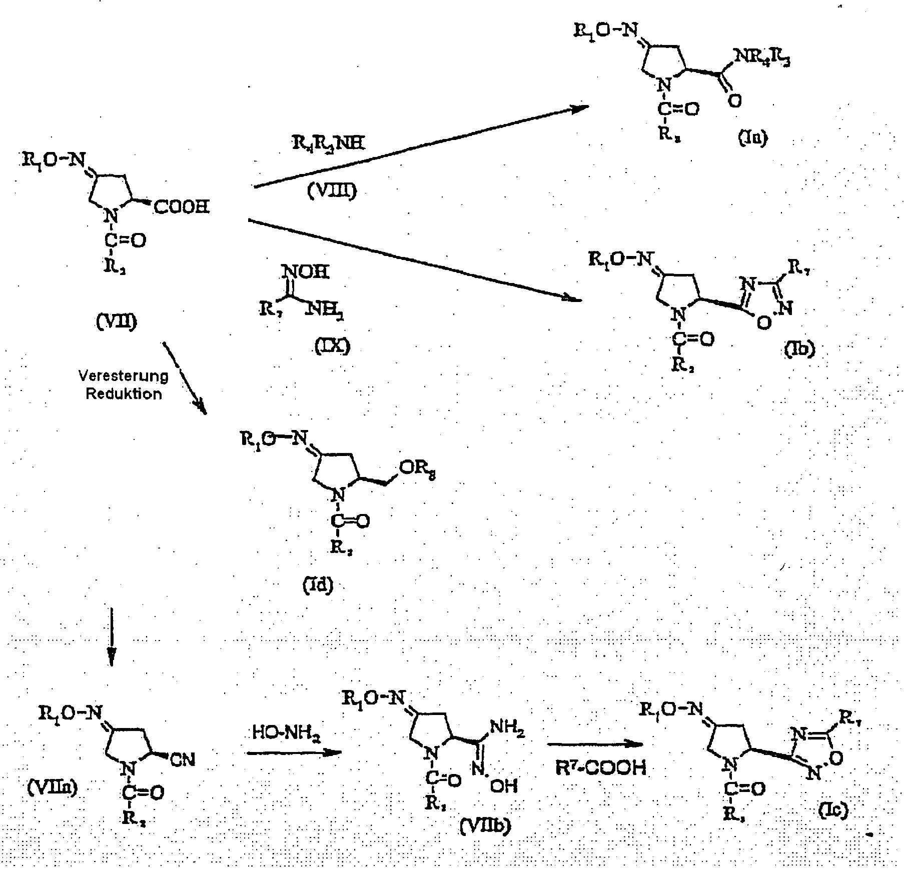

2. Verfahren zur Herstellung einer Verbindung der Formel (I) gemäß Anspruch 1: 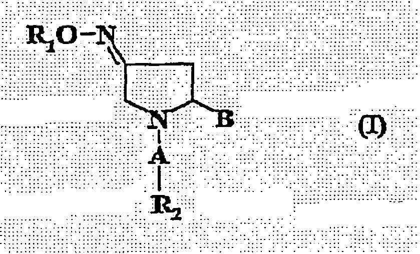 worin
A eine Carbonylgruppe -(C=O)- ist;
B entweder eine Amidogruppe der Formel -(C=O)-NR₃R₄ oder ein Oxadiazolring mit einer der Formeln: 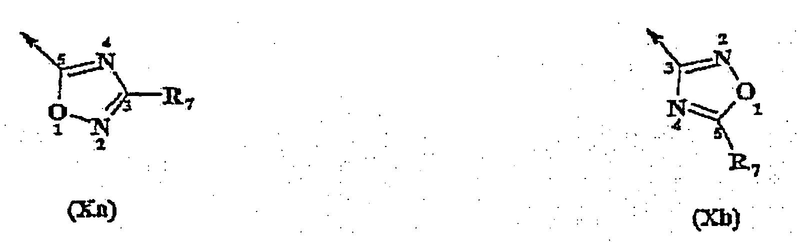 ist;
R₇ ausgewählt ist aus der Gruppe, bestehend aus Wasserstoff, Sulfonyl, Amino, C₁-C₆-Alkyl, C₂-C₆-Alkenyl, C₂-C₆-Alkinyl, wobei die Alkyl-, Alkenyl-, Alkinylketten gegebenenfalls unterbrochen sind durch ein Heteroatom, ausgewählt aus N, O oder S, Aryl, Heteroaryl, gesättigtem oder ungesättigtem 3-8-gliedrigem Cycloalkyl, Heterocycloalkyl, wobei die Cycloalkyl-, Heterocycloalkyl-, Aryl- oder Heteroarylgruppen gegebenenfalls anneliert sind an 1-2 weitere Cycloalkyl-, Heterocycloalkyl-, Aryl- oder Heteroarylgruppen, einem Acylrest, C₁-C₆-Alkylaryl, C₁-C₆-Alkylheteroaryl, C₁-C₆-Alkenylaryl, C₁-C₆-Alkenylheteroaryl, C₁-C₆-Alkinylaryl, C₁-C₆-Alkinylheteroaryl, C₁-C₆-Alkylcycloalkyl, C₁-C₆-Alkylheterocycloalkyl, C₁-C₆-Alkenylcycloalkyl, C₁-C₆-Alkenylheterocycloalkyl, C₁-C₆-Alkinylcycloalkyl, C₁-C₆-Alkinylheterocycloalkyl, Alkoxycarbonyl, Aminocarbonyl, C₁-C₆-Alkylcarboxy, C₁-C₆-Alkylacyl, C₁-C₆-Alkylacyloxy, C₁-C₆-Alkylalkoxy, C₁-C₆-Alkylalkoxycarbonyl, C₁-C₆-Alkylaminocarbonyl, C₁-C₆-Alkylacylamino, C₁-C₆-Alkylureido, C₁-C₆-Alkylamino, C₁-C₆-Alkylammonium, C₁-C₆-Alkylsulfonyloxy, C₁-C₆-Alkylsulfonyl, C₁-C₆-Alkylsulfinyl, C₁-C₆-Alkylsulfanyl, C₁-C₆-Alkylsulfonylamino, C₁-C₆-Alkylaminosulfonyl, Hydroxy, Halogen und Cyano;
R₁ H oder ein C₁-C₆-Alkyl ist;
R₂ ausgewählt ist aus der Gruppe, bestehend aus Aryl, Heteroaryl und gesättigtem oder ungesättigtem 3-8-gliedrigem Cycloalkyl;
R₃ und R₄ unabhängig voneinander ausgewählt sind aus der Gruppe, bestehend aus Wasserstoff, C₁-C₆-Alkyl, C₂-C₆-Alkenyl, C₂-C₆-Alkinyl, Alkoxy, Sulfanyl, Acyl, Alkoxycarbonyl, Aminocarbonyl, gesättigtem oder ungesättigtem 3-8-gliedrigem Cycloalkyl, das 1-3 Heteroatome, ausgewählt aus N, O, S, enthalten kann, Aryl, Heteroaryl, C₁-C₆-Alkylaryl und C₁-C₆-Alkylheteroaryl;
wobei das Verfahren die nachfolgenden Schritte umfasst:
**Schritt 1:** Überführung des Pyrrolidins der Formel (II) in ein Acylderivat der Formel (IV) unter Verwendung eines Acylierungsmittels (III): 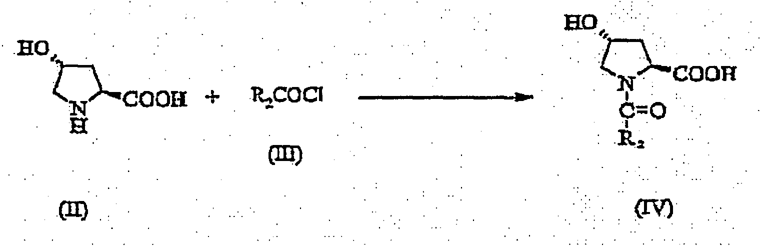
**Schritt 2:** Oxidation des Acylderivat (IV) mit einem Oxidationsmittel, unter Erhalt eines Pyrrolidons der Formel (V): 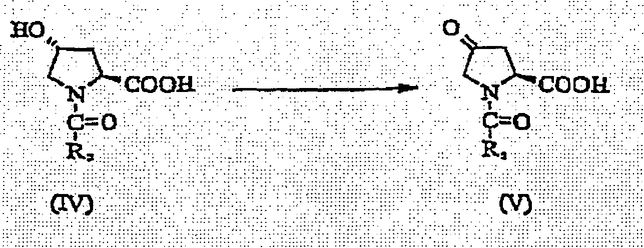
**Schritt 3:** Überführung des Pyrrolidons der Formel (V) in Verbindung (VII) unter Verwendung eines geeigneten Alkoxylamins, Aryloxylamins oder Hydroxylamins der allgemeinen Formel (VI): 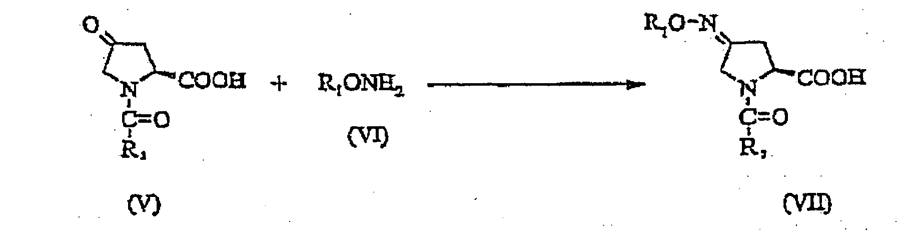
**Schritt 4:** Überführung der Verbindung (VII) mit einem Amin der allgemeinen Formel (VIII) oder einem N-Hydroxyamidin der allgemeinen Formel (IX) unter Erhalt der Verbindungen (Ia) und (Ib) oder Überführung der Verbindung (VII) zunächst in Nitril (VIIa), welches anschließend überführt wird in das Hydroxyamidin (VIIb), welches anschließend umgesetzt wird mit einer Carbonsäure R⁷-COOH, um die Verbindung (Ic) zu erhalten:

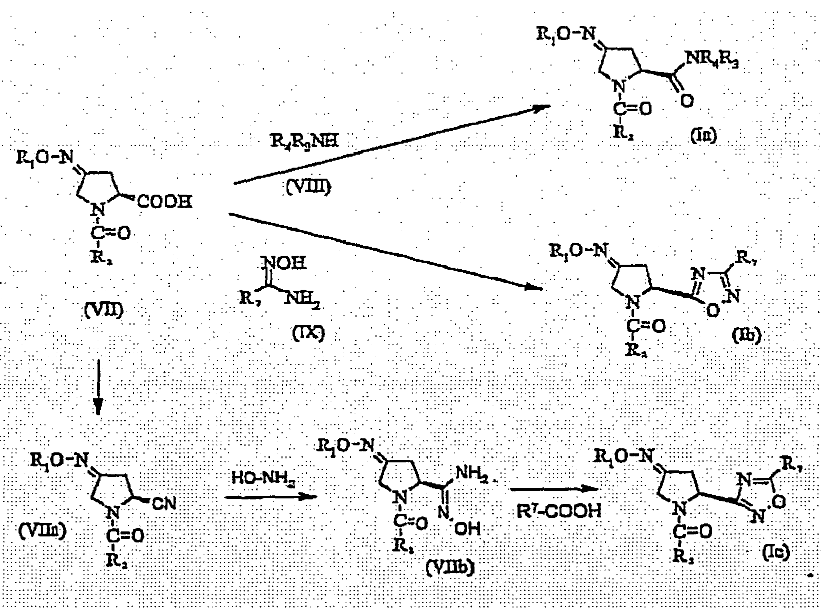

3. Verfahren nach Anspruch 1 oder 2, wobei das Acylchlorid in Schritt 1 1'1-Biphenyl-4-carbonylchlorid oder 2'-Methyl-1',1-biphenyl-4-carbonylchlorid ist.

4. Verfahren nach einem der Ansprüche 1 bis 3, wobei das Oxidationsmittel in Schritt 2 ein Pyridin-Schwefeltrioxid-Komplex (Py-SO₃) in Kombination mit DMSO ist.

5. Verfahren nach einem der Ansprüche 2 bis 4, wobei die Umsetzung in Gegenwart von Triethylamin erfolgt.

6. Verfahren nach einem der Ansprüche 1 bis 5, wobei das Alkoxylamin in Schritt 3 O-Methylhydroxylamin-Hydrochlorid ist.

7. Verfahren nach einem der Ansprüche 1 bis 6, wobei R₁ eine Methylgruppe und R₂ ein Biphenyl ist.

8. Verfahren nach einem der Ansprüche 1 bis 7, wobei B eine Amidogruppe der Formel - (C=O) NHR₅ ist, worin R₅ eine C₁-C₆-Alkylarylgruppe ist.

9. Verfahren nach Anspruch 8, wobei R₅ eine Phenylethylgruppe ist, welche mit einer Amino- oder Hydroxygruppe substituiert ist.

10. Verfahren nach einem der Ansprüche 1 bis 7, wobei B ein 1,2,4-Oxadiazolsubstituent 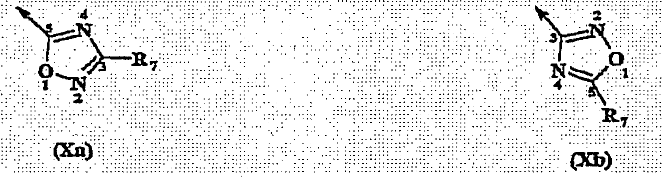 ist, worin R₇ ein C₁-C₆-Alkyl oder ein Cycloalkyl ist, gegebenenfalls enthaltend 1 oder 2 Heteroatome.

11. Verfahren nach einem der Ansprüche 1, 3, 4 oder 6 bis 7, wobei B -(CH₂)ₙ-X-R₈ ist, worin X O ist, R₈ Wasserstoff ist, und n gleich 1 ist.

12. Verfahren nach einem der Ansprüche 1 bis 11, wobei die Verbindung ausgewählt ist aus der Gruppe, bestehend aus:
(2*S*, 4*E* und 4*Z*)-*N*-[(2*S*)-2-Hydroxy-2-phenylethyl]-4-(methoxyimino)-1-[(2'-methyl[1,1'-biphenyl]-4-yl)carbonyl]-2-pyrrolidincarboxamid,
(3*E*,5*S*)-1-([1,1'-Biphenyl]-4-ylcarbonyl)-5-[3-(2-hydroxyethyl)-1,2,4-oxadiazol-5-yl]-3-pyrrolidinon-*O-*methyloxim,
(3*Z*,5*S*)-1-([1,1'-Biphenyl]-4-ylcarbonyl)-5-[3-(2-hydroxyethyl)-1,2,4-oxadiazol-5-yl]-3-pyrrolidon-*O*-methyloxim,
(3*E*,5*S*)-5-[3-(2-Hydroxyethyl)-1,2,4-oxadiazol-5-yl]-1-[(2'-methylbiphenyl-4-yl)carbonyl]pyrrolidin-3-on-O-methyloxim,
(3*Z*,5*S*)-5-[3-(2-Hydroxyethyl)-1,2,4-oxadiazol-5-yl]-1-[(2'-methylbiphenyl-4-yl)carbonyl]pyrrolidin-3-on-*O*-methyloxim,
(3*EZ*,5*S*)-1-([1,1'-Biphenyl]-4-ylcarbonyl)-5-{5-[(dimethylamino)methyl]-1,2,4-oxadiazol-3-yl}-3-pyrrolidinon-*O*-methyloxim,
(3*Z*, 5*S*)-1-([1,1'-Biphenyl]-4-ylcarbonyl)-5-{5-[(dimethylamino)methyl]-1,2,4-oxadiazol-3-yl}-3-pyrrolidinon-O-methyloxim,
(3*E*, 5*S*)-1-([1,1'-Biphenyl]-4-ylcarbonyl)-5-{5-[(dimethylamino)methyl]-1,2,4-oxadiazol-3-yl}-3-pyrrolidinon-O-methyloxim,
(3*EZ*, 5*S*)-5-{5-[(Dimethylamino)methyl]-1,2,4-oxadiazol-3-yl}-1-[(2'-methylbiphenyl-4-yl)carbonyl]pyrrolidin-3-on-O-methyloxim,
(3*Z*,5*S*)-5-{5-[(Dimethylamino)methyl]-1,2,4-oxadiazol-3-yl)-1-[(2'-methylbiphenyl-4-yl)carbonyl]pyrrolidin-3-on-0-methyloxim,
(3E,5S)-5-{5-[(Dimethylamino)methyl]-1,2,4-oxadiazol-3-yl}-1-[(2'-methylbiphenyl-4-yl)carbonyl]pyrrolidin-3-on-*O*-methyloxim und
(3*Z*/*E*, 5*S*)-1-(Biphenyl-4-yl-carbonyl)-5-hydroxymethyl)pyrrolidin-3-on- O-methyloxim.

## Revendications

1. Procédé de préparation d'un composé de formule (1) : 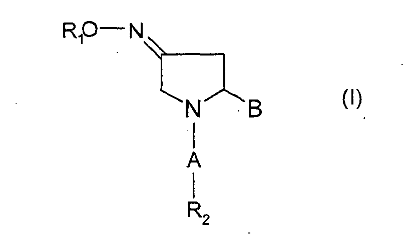 dans laquelle
- A représente un groupe carbonyle -(C=O)-;
- B représente une entité choisie dans l'ensemble formé par un cycle de type oxadiazole, un groupe amido de formule -(C=O)-NR₃R₄, et un groupe de formule -(CH₂)ₙ-X-R₈, le cycle de type oxadiazole ayant l'une des formules suivantes : 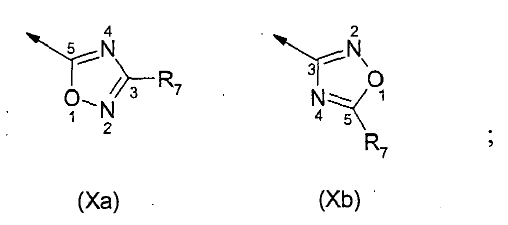
- R₁ représente un atome d'hydrogène ou un groupe alkyle en C₁₋₆;
- R₂ représente une entité choisie parmi les groupes aryle et hétéroaryle et les groupes cycloalkyle saturés ou insaturés, comportant de 3 à 8 chaînons ;
- R₃ et R₄ représentent chacun, indépendamment, une entité choisie dans l'ensemble formé par un atome d'hydrogène et les groupes alkyle en C₁₋₆, alcényle en C₂₋₆, alcynyle en C₂₋₆, alcoxy, sulfanyle, acyle, alcoxycarbonyle, aminocarbonyle, cycloalkyle saturés ou insaturés, comportant de 3 à 8 chaînons et pouvant comporter 1 à 3 hétéroatomes choisis parmi les atomes d'azote, d'oxygène et de soufre, aryle, hétéroaryle, (alkyle en C₁₋₆)-aryle et (alkyle en C₁₋₆)-hétéroaryle ;
- X représente un atome d'oxygène ou une entité symbolisée par NR₉ ;
- R₈ représente une entité choisie dans l'ensemble formé par un atome d'hydrogène et les groupes alkyle en C₁₋₆, (alkyle en C₁₋₆)-aryle, hétéroaryle, (alkyle en C₁₋₆)-hétéroaryle, alcényle en C_{2-6'} (alcényle en C₂₋₆)-aryle, (alcényle en C₂₋₆)-hétéroaryle, alcynyle en C₂₋₆, (alcynyle en C₂₋₆)-aryle, (alcynyle en C₂₋₆)-hétéroaryle, cycloalkyle en C₃₋₈, hétérocycloalkyle, (alkyle en C₁₋₆)-cycloalkyle, (alkyle en C₁₋₆)-hétérocycloalkyle, (alkyle en C₁₋₆)-carboxy, acyle, (alkyle en C₁₋₆)-acyle, (alkyle en C₁₋₆)-acyloxy, (alkyle en C₁₋₆)-alcoxy, alcoxycarbonyle, (alkyle en C₁₋₆)-alcoxycarbonyle, aminocarbonyle, (alkyle en C₁₋₆)-aminocarbonyle, (alkyle en C₁₋₆)-acylamino, (alkyle en C₁₋₆)-uréido, amino, (alkyle en C₁₋₆)-amino, sulfonyloxy, (alkyle en C₁₋₆)-sulfonyloxy, sulfonyle, (alkyle en C₁₋₆)-sulfonyle, sulfinyle, (alkyle en C₁₋₆)-sulfinyle, (alkyle en C₁₋₆)-sulfanyle et (alkyle en C₁₋₆)-sulfonylamino ;
- R₇ représente une entité choisie dans l'ensemble formé par un atome d'hydrogène, les groupes sulfonyle, amino, alkyle en C₁₋₆, alcényle en C₂₋₆, alcynyle en C₂₋₆, les chaînes de ces groupes alkyle, alcényle ou alcynyle pouvant en option comporter, inséré dedans, un hétéroatome choisi parmi les atomes d'azote, d'oxygène et de soufre, aryle, hétéroaryle, cycloalkyle saturés ou insaturés, comportant 3 à 8 chaînons, hétérocycloalkyle, lesquels groupes cycloalkyle, hétérocycloalkyle, aryle et hétéroaryle peuvent, en option, être condensés avec un ou deux autres groupes cycloalkyle, hétérocycloalkyle, aryle ou hétéroaryle, acyle, (alkyle en C₁₋₆)-aryle, (alkyle en C₁₋₆)-hétéroaryle, (alcényle en C₂₋₆)-aryle, (alcényle en C₂₋₆)-hétéroaryle, (alcynyle en C₂₋₆)-aryle, (alcynyle en C₂₋₆)-hétéroaryle, (alkyle en C₁₋₆)-cycloalkyle, (alkyle en C₁₋₆)-hétérocycloalkyle, (alcényle en C₂₋₆)-cycloalkyle, (alcényle en C₂₋₆)-hétérocycloalkyle, (alcynyle en C₂₋₆)-cycloalkyle, (alcynyle en C₂₋₆)-hétérocycloalkyle, alcoxycarbo-nyle, aminocarbonyle, (alkyle en C₁₋₆)-carboxy, (alkyle en C₁₋₆)-acyle, (alkyle en C₁₋₆)-acyloxy, (alkyle en C₁₋₆)-alcoxy, (alkyle en C₁₋₆)-alcoxy- carbonyle, (alkyle en C₁₋₆)-aminocarbonyle, (alkyle en C₁₋₆)-acylamino, (alkyle en C₁₋₆)-uréido, (alkyle en C₁₋₆)-amino, (alkyle en C₁₋₆)-ammo-nium, (alkyle en C₁₋₆)-sulfonyloxy, (alkyle en C₁₋₆)-sulfonyle, (alkyle en C₁₋₆)-sulfinyle, (alkyle en C₁₋₆)-sulfanyle, (alkyle en C₁₋₆)-sulfonyl-amino, (alkyle en C₁₋₆)-aminosulfonyle, hydroxyle et cyano, et les atomes d'halogène ;
- R₉ représente une entité choisie dans l'ensemble formé par un atome d'hydrogène et les groupes alkyle en C₁₋₆, aryle, hétéroaryle, (alkyle en C₁₋₆)-aryle et (alkyle en C₁₋₆)-hétéroaryle ;
- les entités représentées par R₈ et R₉ pouvant aussi former, conjointement avec l'atome d'azote auquel elles sont liées, un groupe hétérocycloalkyle saturé ou insaturé, comportant 5 à 8 chaînons ;
- et n représente un nombre entier valant de 1 à 3 ;
lequel procédé comporte les étapes suivantes :
Etape 1 :
conversion du dérivé de pyrrolidine de formule (II) en un dérivé acylé de formule (IV), au moyen d'un agent d'acylation de formule (III) : 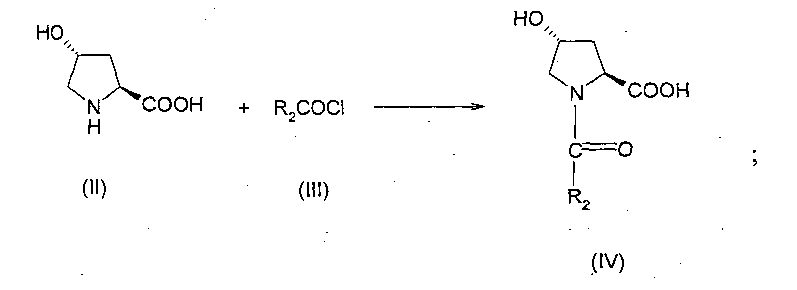
Etape 2 :
oxydation du dérivé acylé de formule (IV) au moyen d'un agent oxydant, ce qui donne un dérivé de pyrrolidinone de formule (V) : 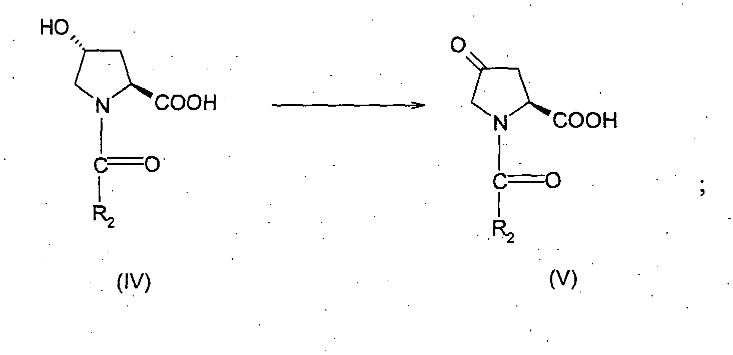
Etape 3 :
conversion de ce dérivé de pyrrolidinone de formule (V) en un composé de formule (VII), au moyen d'hydroxylamine ou d'une alcoxylamine ou aryloxylamine appropriée, de formule générale (VI) : 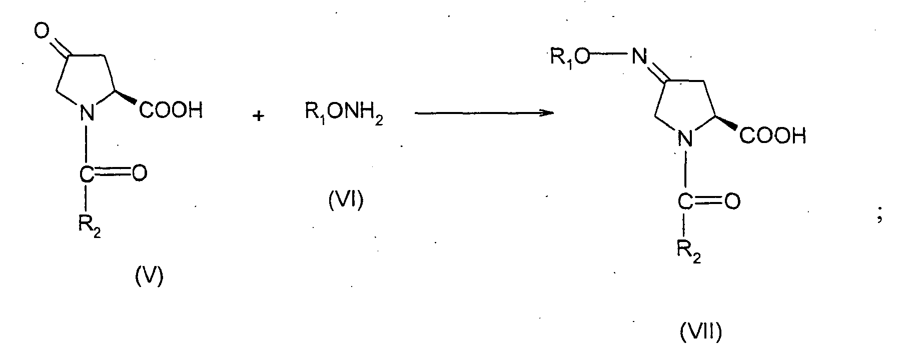
Etape 4:
conversion de ce composé de formule (VII) au moyen d'une amine de formule générale (VIII) ou d'une N-hydroxy-amidine de formule générale (IX), ce qui donne un composé de formule (Ia) ou (Ib),
ou conversion de ce composé de formule (VII), d'abord en un nitrile de formule (VIIa) qui est ensuite converti en une hydroxyamidine de formule (VIIb) qu'on fait réagir avec un acide carboxylique de formule R₇-COOH, ce qui donne un composé de formule (Ic),
ou d'abord estérification de ce composé de formule (VII), puis réduction du produit obtenu, respectivement au moyen d'un agent approprié d'estérification ou de réduction, ce qui donne un composé de formule (Id) :

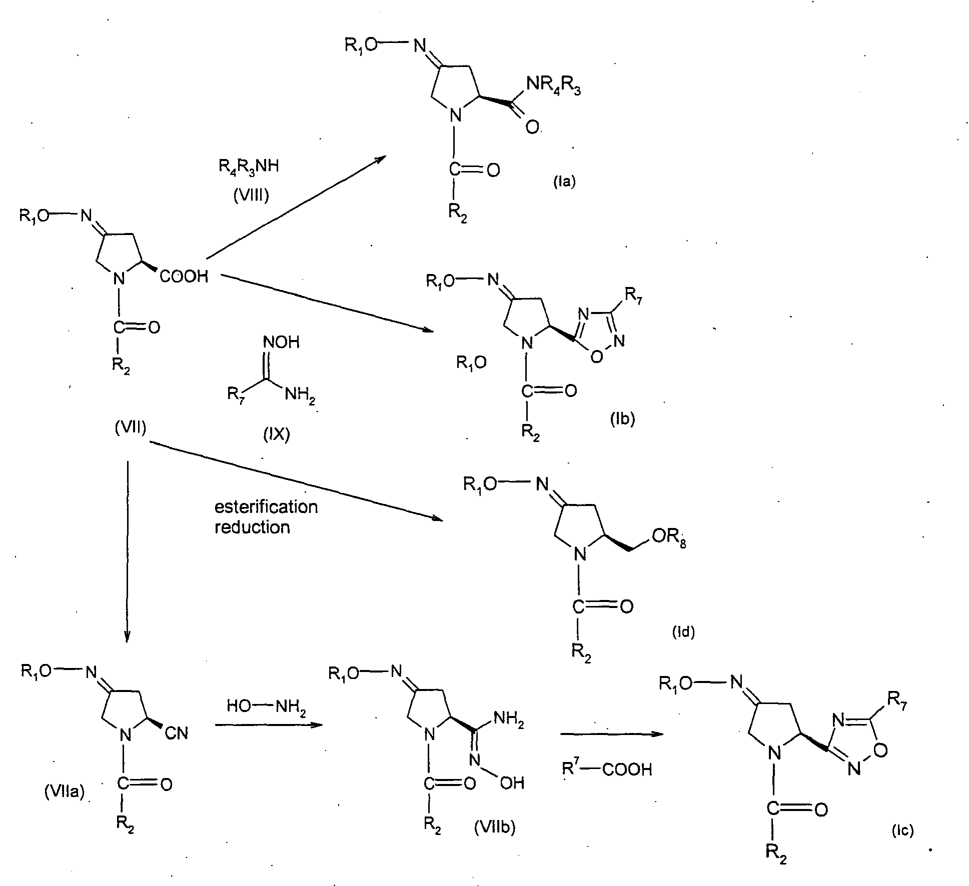

2. Procédé, conforme à la revendication 1, de préparation d'un composé de formule (I) : 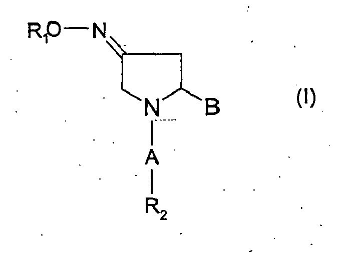 dans laquelle
- A représente un groupe carbonyle -(C=O)- ;
- B représente soit un groupe amido de formule -(C=O)-NR₃R₄, soit un cycle de type oxadiazole ayant l'une des formules suivantes : 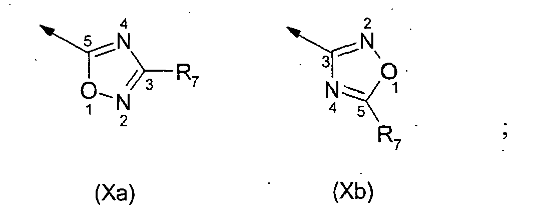
- R₇ représente une entité choisie dans l'ensemble formé par un atome d'hydrogène, les groupes sulfonyle, amino, alkyle en C₁₋₆, alcényle en C₂₋₆, alcynyle en C₂₋₆, les chaînes de ces groupes alkyle, alcényle ou alcynyle pouvant en option comporter, inséré dedans, un hétéroatome choisi parmi les atomes d'azote, d'oxygène et de soufre, aryle, hétéroaryle, cycloalkyle saturés ou insaturés, comportant 3 à 8 chaînons, hétérocycloalkyle, lesquels groupes cycloalkyle, hétérocycloalkyle, aryle et hétéroaryle peuvent, en option, être condensés avec un ou deux autres groupes cycloalkyle, hétérocycloalkyle, aryle ou hétéroaryle, acyle, (alkyle en C₁₋₆)-aryle, (alkyle en C₁₋₆)-hétéroaryle, (alcényle en C₂₋₆)-aryle, (alcényle en C₂₋₆)-hétéroaryle, (alcynyle en C₂₋₆)-aryle, (alcynyle en C₂₋₆)-hétéroaryle, (alkyle en C₁₋₆)-cycloalkyle, (alkyle en C₁₋₆)-hétérocycloalkyle, (alcényle en C₂₋₆)-cycloalkyle, (alcényle en C₂₋₆)-hétérocycloalkyle, (alcynyle en C₂₋₆)-cycloalkyle, (alcynyle en C₂₋₆)-hétérocycloalkyle, alcoxycarbo-nyle, aminocarbonyle, (alkyle en C₁₋₆)-carboxy, (alkyle en C₁₋₆)-acyle, (alkyle en C₁₋₆)-acyloxy, (alkyle en C₁₋₆)-alcoxy, (alkyle en C₁₋₆)-alcoxy- carbonyle, (alkyle en C₁₋₆)-aminocarbonyle, (alkyle en C₁₋₆)-acylamino, (alkyle en C₁₋₆)-uréido, (alkyle en C₁₋₆)-amino, (alkyle en C₁₋₆)-ammo-nium, (alkyle en C₁₋₆)-sulfonyloxy, (alkyle en C₁₋₆)-sulfonyle, (alkyle en C₁₋₆)-sulfinyle, (alkyle en C₁₋₆)-sulfanyle, (alkyle en C₁₋₆)-sulfonyl-amino, (alkyle en C₁₋₆)-aminosulfonyle, hydroxyle et cyano, et les atomes d'halogène;
- R₁ représente un atome d'hydrogène ou un groupe alkyle en C₁₋₆;
- R₂ représente une entité choisie parmi les groupes aryle et hétéroaryle, et les groupes cycloalkyle saturés ou insaturés, comportant de 3 à 8 chaînons ;
- et R₃ et R₄ représentent chacun, indépendamment, une entité choisie dans l'ensemble formé par un atome d'hydrogène et les groupes alkyle en C₁₋₆, alcényle en C₂₋₆, alcynyle en C₂₋₆, alcoxy, sulfanyle, acyle, alcoxycarbonyle, aminocarbonyle, cycloalkyle saturés ou insaturés, comportant de 3 à 8 chaînons et pouvant comporter 1 à 3 hétéroatomes choisis parmi les atomes d'azote, d'oxygène et de soufre, aryle, hétéroaryle, (alkyle en C₁₋₆)-aryle et (alkyle en C₁₋₆)-hétéroaryle ;
lequel procédé comporte les étapes suivantes :
Etape 1 :
conversion du dérivé de pyrrolidine de formule (II) en un dérivé acylé de formule (IV), au moyen d'un agent d'acylation de formule (III) : 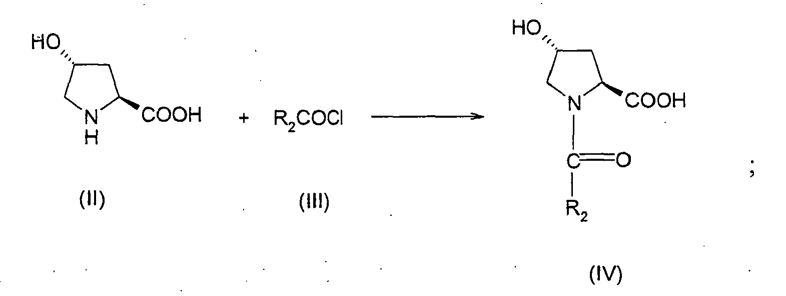
Etape 2 :
oxydation du dérivé acylé de formule (IV) au moyen d'un agent oxydant, ce qui donne un dérivé de pyrrolidinone de formule (V) : 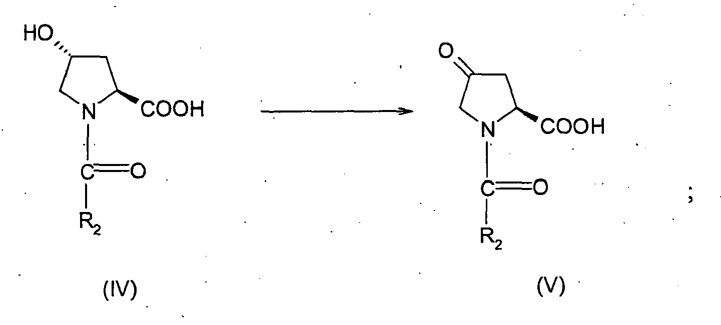
Etape 3 :
conversion de ce dérivé de pyrrolidinone de formule (V) en un composé de formule (VII), au moyen d'hydroxylamine ou d'une alcoxylamine ou aryloxylamine appropriée, de formule générale (VI) : 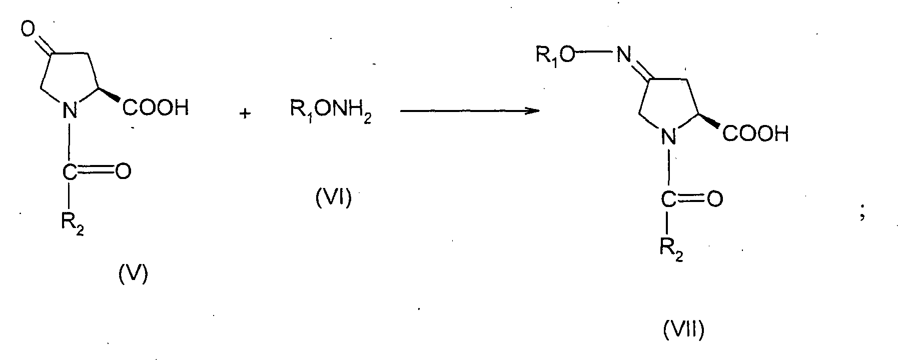
Etape 4 :
conversion de ce composé de formule (VII) au moyen d'une amine de formule générale (VIII) ou d'une N-hydroxy-amidine de formule générale (IX), ce qui donne un composé de formule (Ia) ou (Ib),
ou conversion de ce composé de formule (VII), d'abord en un nitrile de formule (VIIa) qui est ensuite converti en une hydroxyamidine de formule (VIIb) qu'on fait réagir avec un acide carboxylique de formule R₇-COOH, ce qui donne un composé de formule (Ic) :

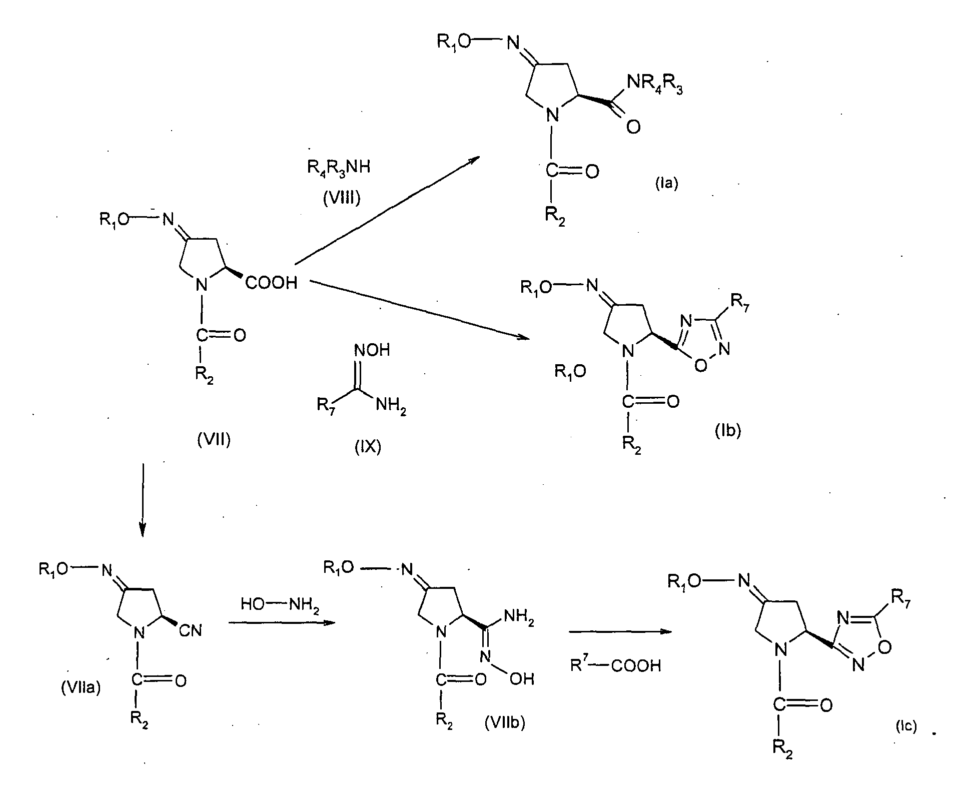

3. Procédé conforme à la revendication 1 ou 2, dans lequel le chlorure d'acyle employé dans l'étape 1 est du chlorure de 1,1'-biphényle-4-carbonyle ou du chlorure de 2'-méthyl1,1'-biphényle-4-carbonyle.

4. Procédé conforme à l'une des revendications 1 à 3, dans lequel l'agent d'oxydation employé dans l'étape 2 est du complexe de pyridine et de trioxyde de soufre (Py-SO₃) associé à du diméthylsulfoxyde (DMSO).

5. Procédé conforme à l'une des revendications 2 à 4, dans lequel la réaction est effectuée en présence de triéthylamine.

6. Procédé conforme à l'une des revendications 1 à 5, dans lequel l'alcoxylamirie employée dans l'étape 3 est du chlorhydrate de O-méthylhydroxylamine.

7. Procédé conforme à l'une des revendications 1 à 6, dans lequel R₁ représente un groupe méthyle et R₂ représente un groupe biphénylyle.

8. Procédé conforme à l'une des revendications 1 à 7, dans lequel B représente un groupe amido de formule -(C=O)-NHR₅ où R₅ représente un groupe (alkyle en C₁₋₆)-aryle.

9. Procédé conforme à la revendication 8, dans lequel R₅ représente un groupe phényl-éthyle porteur d'un substituant amino ou hydroxy.

10. Procédé conforme à l'une des revendications 1 à 7, dans lequel B représente un groupe 1,2,4-oxadiazolyle de formule 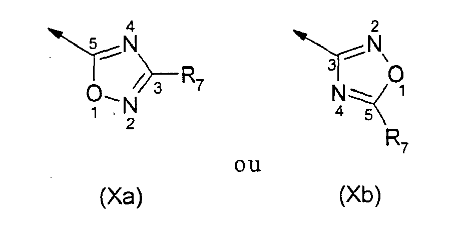 où R₇ représente un groupe alkyle en C₁₋₆ ou cycloalkyle comportant, en option, 1 ou 2 hétéroatomes.

11. Procédé conforme à l'une des revendications 1, 3, 4, 6 et 7, dans lequel B représente un groupe de formule -(CH₂)ₙ-X-R₈ où X représente un atome d'oxygène, R₈ représente un atome d'hydrogène et l'indice n vaut 1.

12. Procédé, conforme à l'une des revendications 1 à 11, de préparation d'un composé choisi dans l'ensemble formé par les suivants :
(2S,4E/Z)-N-[(2S)-2-hydroxy-2-phényl-éthyl]-4-méthoxyimino-1-[(2'-méthyl-1,1'-biphényl-4-yl)carbonyl]pyrrolidine-2-carboxamide
(3E,5S)-O-méthyl-oxime de 1-[(1,1'-biphényl-4-yl)carbonyl]-5-[3-(2-hydroxyéthyl)-1,2,4-oxadiazol-5-yl]pyrrolidin-3-one
(3*Z*,5S)-O-méthyl-oxime de 1-[(1;1'-biphényl-4-yl)carbonyl]-5-[3-(2-hydroxyéthyl)-1,2,4-oxadiazol-5-yl]pyrrolidin-3-one
(3E,SS)-O-méthyl-oxime de 5-[3-(2-hydroxyéthyl)-1,2,4-oxadiazol-5-yl]-1-[(2'-méthyl-biphényl-4-yl)carbonyl]pyrrolidin-3-one
(3Z,5S)-O-méthyl-oxime de 5-[3-(2-hydroxyéthyl)-1,2,4-oxadiazol-5-yl]-1-[(2'-méthyl-biphényl-4-yl)carbonyl]pyrrolidin-3-one
(3E/Z,5S)-O-méthyl-oxime de 1-[(1,1'-biphényl-4-yl)carbonyl]-5-[5-diméthylamino-méthyl)-1,2,4-oxadiazol-3-yl]pyrrolidin-3-one
(3Z,5S)-O-méthyl-oxime de 1-[(1,1'-biphényl-4-yl)carbonyl]-5-[5-diméthylamino-méthyl)-1,2,4-oxadiazol-3-yl]pyrrolidin-3-one
(3E,5S)-O-méthyl-oxime de 1-[(1,1'-biphényl-4-yl)carbonyl]-5-[5-diméthylamino-méthyl)-1,2,4-oxadiazol-3-yl]pyrrolidin-3-one
(3E/Z,5S)-O-méthyl-oxime de 5-[5-(diméthylaminométhyl)-1,2,4-oxadiazol-3-yl]-1-[(2'-méthyl-biphényl-4-yl)carbonyl]pyrrolidin-3-one
(3Z,5S)-O-méthyl-oxime de 5-[5-(diméthylaminométhyl)-1,2,4-oxadiazol-3-yl]-1-[(2'-méthyl-biphényl-4-yl)carbonyl]pyrrolidin-3-one
(3E,5S)-O-méthyl-oxime de 5-[5-(diméthylaminométhyl)-1,2,4-oxadiazol-3-yl]-1-[(2'-méthyl-biphényl-4-yl)carbonyl]pyrrolidin-3-one
(3E/Z,5S)-O-méthyl-oxime de 1-[(biphényl-4-yl)carbonyl]-5-hydroxyméthyl-pyrrolidin-3-one
